(19) Europäisches Patentamt
European Patent Office
Office européen des brevets

(11) EP 1 010 692 A1

(12) EUROPEAN PATENT APPLICATION
published in accordance with Art. 158(3) EPC

(43) Date of publication:
**21.06.2000 Bulletin 2000/25**

(21) Application number: **98929829.4**

(22) Date of filing: **03.07.1998**

(51) Int. Cl.$^7$: **C07D 213/89**, C07D 239/26, C09K 19/24, C09K 19/42, G02F 1/13

(86) International application number:
**PCT/JP98/03012**

(87) International publication number:
**WO 99/01433 (14.01.1999 Gazette 1999/02)**

(84) Designated Contracting States:
**DE FR GB**

(30) Priority: **04.07.1997 JP 19478097**

(71) Applicant: **CHISSO CORPORATION
Osaka-shi, Osaka-fu 530-0005 (JP)**

(72) Inventors:
- **BERNHARD, Henry
D-06120 Halle (DE)**
- **KRESSE, Horst
D-06114 Halle (DE)**
- **DEMUS, Dietrich
D-06118 Halle (DE)**
- **TAKEUCHI, Hiroyuki
Ichihara-shi, Chiba 290-0022 (JP)**
- **MIYAZAWA, Kazutoshi
Ichihara-shi, Chiba 290-0158 (JP)**
- **HISATSUNE, Yasusuke
Ichihara-shi, Chiba 299-0111 (JP)**
- **TAKESHITA, Fusayuki
Kimitsu-shi, Chiba 299-1132 (JP)**
- **NAKAGAWA, Etsuo
Ichihara-shi, Chiba 290-0056 (JP)**
- **MATSUI, Shuichi
Ichihara-shi, Chiba 290-0003 (JP)**

(74) Representative: **HOFFMANN - EITLE
Patent- und Rechtsanwälte
Arabellastrasse 4
81925 München (DE)**

# (54) LIQUID-CRYSTAL COMPOUNDS HAVING LARGE VALUE OF PERMITTIVITY ANISOTROPY, LIQUID-CRYSTAL COMPOSITION CONTAINING THE SAME, AND LIQUID-CRYSTAL DISPLAY ELEMENT MADE USING THE LIQUID-CRYSTAL COMPOSITION

(57) Provided are liquid crystalline compounds having an extremely large dielectric anisotropy value, liquid crystal compositions comprising the compound and having a low driving voltage, and liquid crystal display devices fabricated by using the liquid crystal composition; and the liquid crystalline compounds are expressed by the general formula (1)

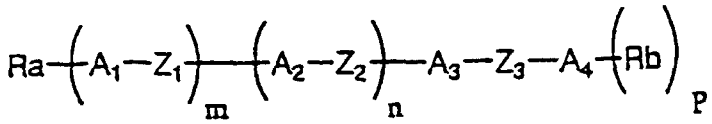

(1)

wherein Ra represents a straight chain or branched alkyl group having 1 to 20 carbon atoms; Rb represents a group selected from Ra, a halogen atom, or cyano group; Rc and Rd each independently represent hydrogen atom or an alkyl group having 1 to 3 carbon atoms; $A_1$, $A_2$, $A_3$, and $A_4$ each independently represent pyridine oxide, pyrimidine dioxide, trans-1,4-cyclohexylene, or 1,4-phenylene provided that at least one of $A_1$, $A_2$, $A_3$, and $A_4$ is pyridine oxide ring or pyrimidine dioxide ring; Re represents hydrogen atom, a halogen atom, or an alkyl group having 1 to 3 carbon atoms; $Z_1$, $Z_2$, and $Z_3$ each independently represent single bond or an alkylene group having 1 to 4 carbon atoms one or more hydrogen atoms in which group may be replaced by halogen atoms; m, n, and p are each independently 0 or 1 provided that p is 0 when $A_4$ is pyridine oxide ring bonded, at its position 4, to $Z_3$; and the atom which constitutes these compounds may be replaced by its isotope.

EP 1 010 692 A1

## Description

### TECHNICAL FIELD

The present invention relates to novel liquid crystalline compounds and liquid crystal compositions. More specifically, the invention relates to liquid crystalline compounds having pyridine oxide ring or pyrimidine dioxide ring, liquid crystal compositions comprising the compound, and liquid crystal display devices fabricated by using the liquid crystal composition.

### BACKGROUND ART

Display devices fabricated by using liquid crystalline compounds are widely employed in displays of computers, monitors, and others. In this specification, the term "liquid crystalline compound" is used as a generic name for the compounds exhibiting a liquid crystal phase and the compounds which do not exhibit a liquid crystal phase but are useful as a component of liquid crystal compositions.
Driving mode of displays which make full use of a liquid crystal composition can broadly be divided into three types of TN mode, STN mode, and TFT mode.
In any driving mode, it is required to lower driving voltage for the purpose of reducing power consumption and decreasing leakage electromagnetic wave. Driving voltage (threshold voltage) is known to be a function of dielectric anisotropy value and elastic constant according to the following equation (M. F. Leslie, Mol. Cryst. Liq. Cryst., 12, 57 (1970)):

$$Vth = \pi(K/\varepsilon_0 \Delta\varepsilon)^{1/2}$$

wherein Vth is a threshold voltage, $\varepsilon_0$ is a dielectric constant in vacuum, K is an elastic constant, and $\Delta\varepsilon$ is a dielectric anisotropy, respectively.
That is, it is understood that in order to lower driving voltage, 1) dielectric anisotropy value is necessary to be increased or 2) elastic constant is required to decrease.
It is generally considered to be difficult to adjust the value of elastic constant and thus principally a means in which the dielectric anisotropy value is increased is adopted to lower driving voltage. Accordingly, novel liquid crystalline compounds particularly having a large dielectric anisotropy value are long-expected.

### DISCLOSURE OF THE INVENTION

In view of the required characteristics described above, an object of the present invention is to provide liquid crystalline compounds having an extremely large dielectric anisotropy value, liquid crystal compositions comprising the compound, and liquid crystal display devices fabricated by using the liquid crystal composition.
As a result of diligent research by the present inventors to solve the problems described above, it has been found out that the compounds having pyridine oxide ring or pyrimidine dioxide ring in the molecule and expressed by the general formula (1) have desired characteristics, leading to the accomplishment of the present invention.

$$Ra-(A_1-Z_1)_m-(A_2-Z_2)_n-A_3-Z_3-A_4-(Rb)_p \qquad (1)$$

wherein Ra represents a straight chain or branched alkyl group having 1 to 20 carbon atoms in which groups any methylene group ($-CH_2-$) may be replaced by -O-, -CO-, -CH=CH-, -C≡C-, -SiRcRd-, cyclopropane-1,2-diyl, bicyclo[1.1.1]pentane-1,3-diyl, or cyclobutane-1,3-diyl, but there is not a case in which -O- continues, and one or more hydrogen atoms in Ra may be replaced by halogen atoms or cyano groups;

Rb represents a group selected from Ra, a halogen atom, or cyano group;
Rc and Rd each independently represent hydrogen atom or an alkyl group having 1 to 3 carbon atoms;
$A_1$, $A_2$, $A_3$, and $A_4$ each independently represent pyridine oxide, pyrimidine dioxide, trans-1,4-cyclohexylene, or 1,4-phenylene provided that at least one of $A_1$, $A_2$, $A_3$, and $A_4$ is pyridine oxide ring or pyrimidine dioxide ring, in these rings any $-CH_2-$ may be replaced by -O-, >CH- may be replaced by >SiRe-, and -CH= may be replaced by -N=, respectively, and one or two hydrogen atoms on the six-membered ring may be replaced by halogen atoms;
Re represents hydrogen atom, a halogen atom, or an alkyl group having 1 to 3 carbon atoms;
$Z_1$, $Z_2$, and $Z_3$ each independently represent single bond or an alkylene group having 1 to 4 carbon atoms one or more hydrogen atoms in which group may be replaced by halogen atoms, and any methylene group ($-CH_2-$) in which alkylene group may be replaced by -O-, -CO-, -CH-CH-, -C≡C-, or $-SiH_2-$, but there is not a case in which -O- continues;
m, n, and p are each independently 0 or 1 provided that p is 0 when $A_4$ is pyridine oxide ring bonded, at its position 4, to $Z_3$; and
the atom which constitutes these compounds may be replaced by its isotope.

In the general formula (1), Ra is a straight chain or branched alkyl group having 1 to 20 carbon atoms. As the straight chain alkyl group, methyl, ethyl, propyl, butyl, pentyl, hexyl, heptyl, decyl, pentadecyl, and icosyl; and as the branched alkyl group, isopropyl, sec-butyl, tert-butyl, 2-methylbutyl, isopentyl, and isohexyl can be mentioned as their examples.
Any methylene group ($-CH_2-$) in these alkyl groups may be replaced by -O-, -CO-, -CH=CH-, -C≡C-, $-SiH_2-$, $-Si(CH_3)_2-$, cyclopropane-1,2-diyl, bicyclo[1.1.1]pentane-1,3-diyl, or cyclobutane-1,3-diyl unless -O- continues.
As examples of these groups, alkoxy groups, alkoxyalkyl groups, alkenyl groups, alkadienyl groups, alkenyloxy groups, alkoxyalkenyl groups, alkynyl groups, alkynyloxy groups, alkoxyalkynyl groups, silanyl groups, alkylsilyl groups, alkoxysilyl groups, alkylsilylalkyl groups, alkoxysilylalkyl groups, alkyldisilanyl groups, alkyldisilanylalkyl groups, and alkyltrisilanyl groups can be mentioned. Besides, one or more hydrogen atoms in these groups may be replaced by halogen atoms, and as their examples, halogen substituted alkyl groups, halogen substituted alkoxy groups, halogen substituted alkenyl groups, and halogen substituted alkynyl groups can be mentioned.
Among these, preferable groups are specifically mentioned as follows:
As the groups in which $-CH_2-$ is replaced by -O-, alkoxy groups such as methoxy, ethoxy, propoxy, butoxy, pentyloxy, and nonyloxy; alkoxyalkyl groups such as methoxymethyl, methoxyethyl, methoxypropyl, methoxybutyl, methoxypentyl, methoxyoctyl, ethoxymethyl, ethoxyethyl, etoxylpropyl, ethoxyhexyl, propoxymethyl, propoxyethyl, propoxypropyl, propoxypentyl, butoxymethyl, butoxyethyl, butoxybutyl, pentyloxymethyl, pentyloxybutyl, hexyloxymethyl, hexyloxyethyl, hexyloxypropyl, heptyloxymethyl, and oxtyloxymethyl; and branched alkoxy groups such as 2-methylpropoxy, 2-methylpentoxy, and 1-methylheptoxymethyl can be mentioned.
As the groups in which $-CH_2-$ is replaced by -CH=CH-, alkenyl groups such as vinyl, propenyl, butenyl, pentenyl, hexenyl, and decenyl; alkoxyalkenyl groups such as methoxypropenyl, ethoxypropenyl, pentyloxypropenyl, methoxybutenyl, ethoxybutenyl, pentyloxybutenyl, methoxypentenyl, propoxypentenyl, methoxyhexenyl, propoxyhexenyl, methoxyheptenyl, and methoxyoctenyl; alkenyloxoy groups such as propenyloxy, butenyloxy, pentenyloxy, octenyloxy, and propenyloxymethyl; groups such as propenyloxyethyl, propenyloxybutyl, butenyloxymethyl, butenyloxyethyl, butenyloxypentyl, pentenyloxymethyl, pentenyloxypropyl, hexenyloxymethyl, hexenyloxyethyl, heptenyloxymethyl, and octenyloxymethyl; and alkadienyl groups such as butadienyl, heptadienyl, hexadienyl, heptadienyl, octadienyl, and icosadienyl can be mentioned.
As the groups in which $-CH_2-$ is replaced by -C≡C-, alkynyl groups such as ethynyl, propynyl, butynyl, pentynyl, and octynyl; alkynyloxy groups such as ethynyloxy, propynyloxy, butynyloxy, pentynyloxy, and tetradecynyloxy; and alkoxyalkynyl groups such as methoxypropynyl, methoxypentynyl, ethoxybutynyl, propoxypropynyl, hexyloxyheptynyl, methoxymethylbutynyl, methoxypropylethynyl, and butoxymethylpropynyl can be mentioned.
As the groups in which $-CH_2-$ is replaced by $-SiH_2-$, alkylsilyl groups such as methylsilyl, ethylsilyl, propylsilyl, butylsilyl, pentylsilyl, and nonylsilyl; alkylsilylalkyl groups such as methylsilylmethyl, methylsilylethyl, methylsilylpropyl, methylsilylbutyl, methylsilylheptyl, ethylsilylmethyl, ethylsilylethyl, ethylsilylpropyl, ethylsilylhexyl, propylsilylmethyl, propylsilylethyl, propylsilylpropyl, butylsilylmethyl, butylsilylethyl, butylsilylpropyl, pentylsilylmethyl, hexylsilylmethyl, hexylsilylethyl, heptylsilylmethyl, and oxtylsilylmethyl; alkoxysilyl groups such as methoxysilyl, ethoxysilyl, propoxysilyl, butoxysilyl, pentyloxysilyl, and oxtyloxysilyl; and silanyl groups such as silanyl, disilanyl, trisilanyl, tetrasilanyl, pentasilanyl, and decasilanyl; alkyldisilanyl groups such as methyldisilanyl, ethyldisilanyl, propyldisilanyl, butyldisilanyl, and pentyldisilanyl; alkyltrisilanyl groups such as methyltrisilanyl, ethyltrisilanyl, propyltrisilanyl, and hexyltrisilanyl; alkyldisilanylalkyl groups such as methyldisilanylmethyl, methyldisilanylethyl, methyldisilanylpentyl, ethyldisilanylmethyl, ethyldisilanylethyl, ethyldisilanylbutyl, ethyldisilanylhexyl, propyldisilanylmethyl, butyldisilanylpentyl, pentyldisilanylmethyl, hexyldisilanylethyl, and heptyldisilanylmethyl; alkyltrisilanylalkyl groups such as methyltrisilanylmethyl, methyltrisilanylpentyl, ethyltrisilanylmethyl, ethyltrisilanylpropyl, propyltrisilanylmethyl, propyltrisilanylbutyl, butyltrisilanylmethyl, pentyltrisilanylmethyl, and hexyltrisilanylmethyl; and groups such as methylnonasilanyl, methylhexasilanylmethyl, ethylheptasilanylmethyl, methyloctasilanylmethyl, 2-fluoroethylsilyl, 3,3-difluoropropylsilyl, and 1,2,3,3-tetrafluoropropylsilyl can be mentioned.
As the groups in which $-CH_2-$ is replaced by -SiRcRd-, groups such as trimethylsilyl, ethyldimethylsilyl, propyldimethylsilyl, and butyldimethylsilyl can be mentioned.

As the groups in which $-CH_2-$ is replaced by -CO-, groups such as methylcarbonyl, ethylcarbonyl, propylcarbonyl, methoxycarbonyl, ethoxycarbonyl, propoxycarbonyl, butoxycarbonyl, heptyloxycarbonyl, 2-oxopropyl, 2-oxobutyl, 3-oxobutyl, 2-oxopentyl, 4-oxopentyl, 3-oxohexyl, 5-oxohexyl, 2-oxoheptyl, 3-oxoheptyl, 6-oxoheptyl, 2-oxooctyl, 4-oxooctyl, 7-oxooctyl, 3-oxononyl, 6-oxononyl, 8-oxononyl, 2-oxodecyl, 5-oxodecyl, and 9-oxodecyl can be mentioned.

Also, hydrogen atom in these groups may be replaced by a halogen atom or cyano group, and specifically, halogen substituted alkyl groups such as fluoromethyl, difluoromethyl, trifluoromethyl, 2-fluoroethyl, 1,2-difluoroethyl, 2-bromo-1,2-difluoroethyl, 3-fluoropropyl, 1,2,3,3-tetrafluoropropyl, 4-fluorobutyl, 1,1,2,4-tetrafluorobutyl, 5-fluoropentyl, 1,1,3,3,3-pentafluoropropyl, 2,3,3,4,5-pentafluoropentyl, 6-fluorohexyl, 2,3,4,6-tetrafluorohexyl, 7-fluoroheptyl, and 8,8-difluorooctyl; halogen substituted alkoxy groups such as difluoromethoxy, trifluoromethoxy, 1,1-difluoroethoxy, 2,2-difluoroethoxy, 2,2,2-trifluoroethoxy, 1,1,2,2-tetrafluoroethoxy, perfluoroethoxy, 1,1,3,3,3-pentafluoropropoxy, 1,1,2,3,3,3-hexafluoropropoxy, and perfluoropropoxy; and halogen substituted alkenyl groups such as 3-fluoropropenyl, 4-fluoro-1-butenyl, 4-fluoro-2-butenyl, 5-fluoro-1-pentenyl, 5-fluoro-2-pentenyl, 5-fluoro-3-pentenyl, 6-fluoro-1-hexenyl, 6-fluoro-3-hexenyl, 7-fluoro-5-heptenyl, 2,2-difluorovinyl, 1,2-difluorovinyl, 2-chloro-2-fluorovinyl, 2-bromo-2-fluorovinyl, 2-fluoro-2-cyanovinyl, 3,3-difluoro-2-propenyl, 3-chloro-3-fluoro-1-propenyl, 2,3-difluoro-1-propenyl, 1,3-difluoro-2-propenyl, 1,3,3-trifluoro-2-propenyl, 1,2,4,4-tetrafluoro-3-butenyl, 5,5-difluoro-4-pentenyl, 3,3-difluorohexenyl, and 8,8-difluoro-7-octenyl can be mentioned.

Among these, alkyl groups, alkoxy groups, alkoxyalkyl groups, alkenyl groups, alkoxyalkenyl groups, alkylsilyl groups, halogen substituted alkyl groups, halogen substituted alkoxy groups, and halogen substituted alkenyl groups are more preferable.

Next, Rb is selected from a group which is selected in turn from Ra; cyano group; and a group of halogen atoms including F, Cl, Br, and I. However, groups or atoms excepting Br and I are preferable from the viewpoint of stability and others.

While $A_1$, $A_2$, $A_3$, and $A_4$ each independently represent pyridine oxide, pyrimidine dioxide, trans-1,4-cyclohexylene, or 1,4-phenylene, at least one of $A_1$, $A_2$, $A_3$, and $A_4$ is pyridine oxide ring or pyrimidine dioxide ring, in these rings any $-CH_2-$ may be replaced by -O-, >CH- may be replaced by >SiRe-, and -CH= may be replaced by -N=, respectively, and one or two hydrogen atoms on the six-membered ring may be replaced by halogen atoms. While piperidinediyl, piperazinediyl, pyridinediyl, pyrazinediyl, pyrimidinediyl, pyridazinediyl, triazinediyl, tetrazinediyl, tetrahydropyrandiyl, dioxanediyl, and silacyclohexanediyl can specifically be mentioned, at least one of them is pyridine oxide ring or pyrimidine dioxide ring.

As preferable groups among them, trans-1,4-cyclohexylene, 1,4-phenylene, and 1,4-phenylene in which one or two hydrogen atoms on the ring are replaced by halogen atoms, i.e., 2-fluoro-1,4-phenylene, 3-fluoro-1,4-phenylene, 2,3-difluoro-1,4-phenylene, 3,5-difluoro-1,4-phenylene, 2-chloro-1,4-phenylene, 3-chloro-1,4-phenylene, 2,3-dichloro-1,4-phenylene, 3,5-dichloro-1,4-phenylene, 2-chloro-3-fluoro-1,4-phenylene, 3-chloro-2-fluoro-1,4-phenylene, and 3-fluoro-5-chlorophenylene; and in the case of the divalent groups of six-membered rings having a hetero- atom, pyridine-2,5-diyl, pyrimidine-2,5-diyl, tetrahydropyran-2,5-diyl, 1,3-dioxane-2,5-diyl, 1,4-dioxane-2,5-diyl, and 1-sila-1,4-cyclohexanediyl can be mentioned. Further, when a cis-trans isomers exist with these six-membered rings, the type of isomerism of the rings is more preferably trans-form.

While $Z_1$, $Z_2$, and $Z_3$ each independently represent single bond or an alkylene group having 1 to 4 carbon atoms one or more hydrogen atoms contained in which group may be replaced by halogen atoms, single bond, ethylene, or butylene is preferable. Besides, methylene group ($-CH_2-$) which constitutes the alkylene group may be replaced by $-SiH_2-$, -O-, -CH=CH-, or -C≡C- but there is not a case in which -O- continues. As such groups, groups, having $-SiH_2-$, such as 1,2-disilanediyl, 1,4-tetrasilanediyl, 1-silaethylene, 2-silaethylene, 1-sila-1,4-butylene, 2-sila-1,4-butylene, and 3-sila-1,4-butylene; groups, having -O-, such as oxymethylene, methylenoxy, 1-oxa-1,4-butylene, 2-oxa-1,4-butylene, 3-oxa-1,4-butylene, 4-oxa-1,4-butylene, and ester bond; groups, having -CH=CH-, such as vinylene, 1-butenylene, 2-butenylene, and 3-butenylene; groups, having -C≡C-, such as ethynylene, 1-butynylene, 2-butynylene, and 3-butynylene; the groups mentioned above in which one or more hydrogen atoms are replaced by halogen atoms, for examples, fluoromethylenoxy, oxyfluoromethylene, difluoromethylenoxy, oxydifluoromethylene, 1,1-difluoroethylene, 2,2-difluoroethylene, 1,2-difluorovinylene, 1-fluorovinylene, 1-bromo-2-fluorovinylene, 1-chloro-2-fluorovinylene, 1,2-difluoro-1-butenylene, 2,3-difluoro-2-butenylene, and 3,4-difluoro-3-butenylene; and other groups such as 3-oxa-1-butenylene and 4-oxa-1-butenylene can preferably be mentioned.

All the compounds of the present invention comprising the combination of groups selected from the Ra to Re, $A_1$ to $A_4$, and $Z_1$ to $Z_3$ have characteristics inherent to the compounds of the present invention and thus they are preferable compounds.

Among such a group of compounds, ones expressed by one of the following general formulas (1-1) to (1-32) can be mentioned as examples of the compounds having particularly preferable characteristics:

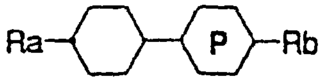 (1-1)

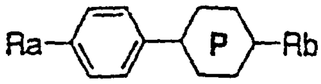 (1-2)

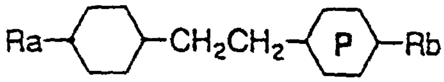 (1-3)

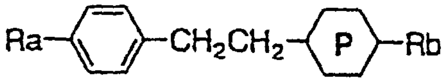 (1-4)

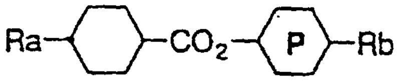 (1-5)

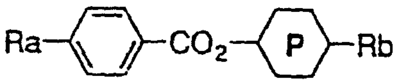 (1-6)

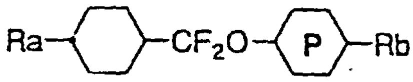 (1-7)

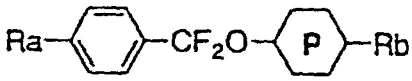 (1-8)

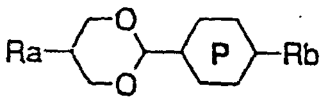 (1-9)

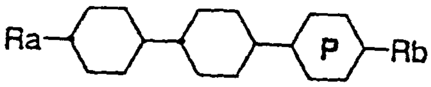 (1-10)

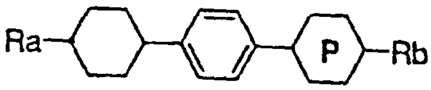 (1-11)

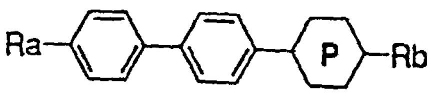 (1-12)

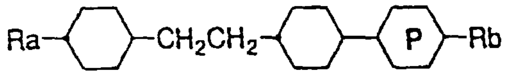 (1-13)

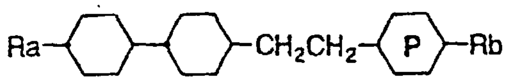 (1-14)

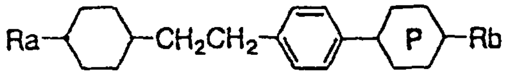 (1-15)

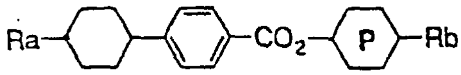 (1-16)

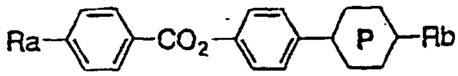 (1-17)

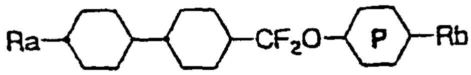 (1-18)

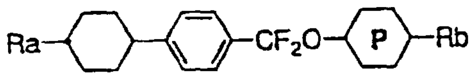 (1-19)

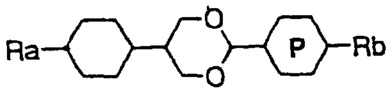 (1-20)

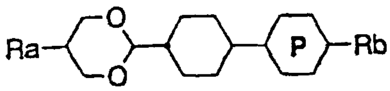 (1-21)

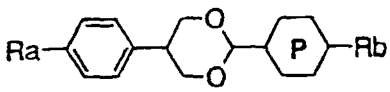 (1-22)

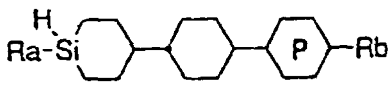 (1-23)

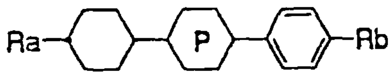 (1-24)

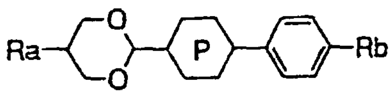 (1-25)

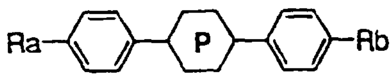 (1-26)

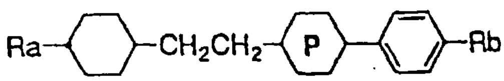 (1-27)

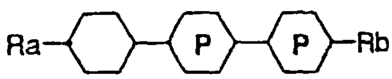 (1-28)

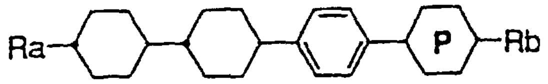
(1-29)

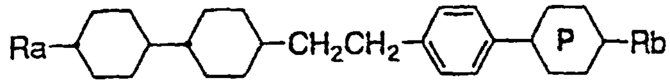
(1-30)

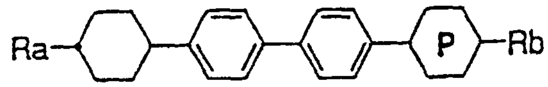
(1-31)

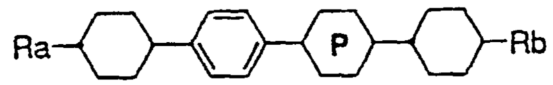
(1-32)

wherein Ra and Rb have the same meaning as described above, and ring P represents pyridine oxide ring or pyrimidine dioxide ring, and hydrogen atom on trans-1,4-cyclohexylene ring, 1,4-phenylene ring, 1,3-dioxane ring, or silacyclohexane ring may be replaced by a halogen atom or cyano group.

Compounds expressed by the general formula (1) can be produced by using known procedures in organic synthetic chemistry in a proper combination. Specifically, they can readily be produced by oxidizing a corresponding pyridine derivative or pyrimidine derivative.

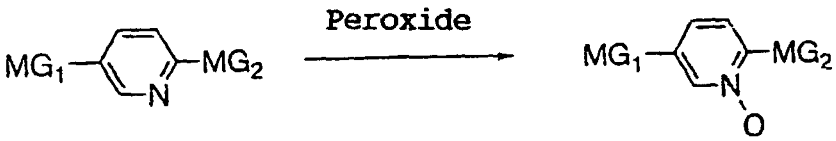


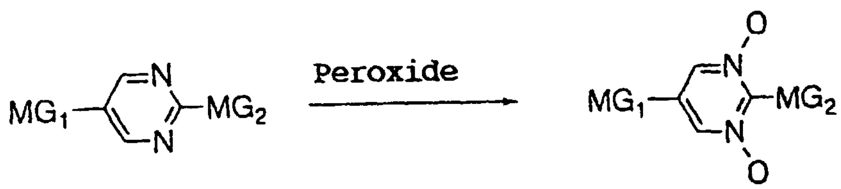


wherein $MG_1$ and $MG_2$ represent organic compound residues (meso-gene).

That is, the compounds of the general formula (1) can be obtained by treating a corresponding pyridine derivative or pyrimidine derivative with a peroxide. AS the peroxide, while the one usually employed is sufficient, for example, hydrogen peroxide, performic acid, peracetic acid, or m-chloroperbenzoic acid can preferably be used.

While the pyridine derivative or pyrimidine derivative of the starting material can be produced according to a method described in known literatures, for instance, they can preferably be produced according to the following literatures:

Inoue et al., EP 239,403
Sugimori et al., EP 194,153
Miyazawa et al., EP 283,326
A. de Meijere et al., DE-OS 3,915,804
A. I. Pavlyuchenko et al., Mol. Cryst. Liq. Cryst., 209, 225 (1995), EP 374,849, PCT-WO 88/07,992
A. Waechtler et al., PCT-WO 89/10,356
D. J. Byron et al., Mol. Cryst. Liq. Cryst., 62, 103 (1980)
E. Poetsch et al., DE-OS 3,736,489
G. W. Gray et al., DE-OS 3,600,052
H. Schlosser et al., DE-OS 4,236,103
J. D. Margerum et al., Mol. Cryst. Liq. Cryst., 122, 97 (1985)

J. Krause et al., DE 3,515,633
M. F. Grebyonkim et al., Mol. Cryst. Liq. Cryst., 129, 245 (1985)
M. P. Burrow et al., Liq. Cryst., 3, 1643 (1988)
M. Schadt et al., EP 589,331
R. A. Champa et al., Mol. Cryst. Liq. Cryst., 19, 233 (1973)
R. Buchecker et al., EP 361,157
R. Dabrowski et al., Mol. Cryst. Liq. Cryst., 107, 411 (1984)
S. M. Kelly et al., Ferroelectrics, 180, 269 (1996), J. Mater. Chem., 3, 953 (1993), Liq. Cryst., 20, 77 (1996)
V. F. Petrov, Mol. Cryst. Liq. Cryst., 265, 47 (1995)
V. Reiffenrath et al., EP 332,024, DE 3,906,059, DE 3,710,069
W. Hemmerling et al., DE-OS 3,827,600

Liquid crystalline compounds of the present invention obtained in such manners exhibit an extremely large dielectric anisotropy value and thus low voltage driving can be actualized. In addition, they are readily mixed with various liquid crystal materials and have a remarkably excellent miscibility even at low temperatures.

Further, these liquid crystalline compounds of the present invention are sufficiently stable physically and chemically under conditions in which liquid crystal display devices are usually employed, and are considerably excellent as component of nematic liquid crystal compositions.

Liquid crystalline compounds of the present invention can preferably be used as component in liquid crystal compositions for TN, STN, TFT, or other display modes.

Further, while the liquid crystalline compounds of the present invention exhibit a negative and extremely large dielectric anisotropy value by selecting proper chemical structure, these compounds can be very preferably used as component of liquid crystal compositions for IPS (in-plane switching) mode or VA (vertical alignment) mode.

Among the compounds expressed by the general formula (1), compounds having two six-membered rings exhibit a comparatively low isotropic phase transition temperature and low viscosity, and compounds having three or four six-membered rings exhibit a high isotropic phase transition temperature and slightly high viscosity. Compounds having cyclohexane ring, dioxane ring, tetrahydropyran ring, or silacyclohexane ring in the molecule exhibit a low $\Delta n$, compounds having cyclohexane ring, silacyclohexane ring, or benzene ring exhibit a low viscosity, compounds having benzene ring, pyridine ring, or pyrimidine ring exhibit a wide temperature range of liquid crystal phase and high $\Delta n$, and compounds having pyridine ring, pyrimidine ring, or dioxane ring exhibit a comparatively high $\Delta\varepsilon$.

By replacing hydrogen atom in the ring structure with fluorine atom, it is possible to obtain a higher $\Delta\varepsilon$ and the miscibility can simultaneously be improved.

Since the compounds in which any one or more of Ra, Rb, and $Z_1$ to $Z_3$ are groups having double bond exhibit a large elastic constant ratio (bend elastic constant/splay elastic constant) and low viscosity, liquid crystal compositions of which the change of the transmittance in T-V curve is steep can be produced and display devices of high contrast can be provided when the compounds are used as component of compositions for STN.

Compounds in which any one or more of Ra, Rb, and $Z_1$ to $Z_3$ are groups having triple bond exhibit a high $\Delta n$ and low viscosity and the compounds having a substituent containing dihydrosilyl group ($-SiH_2-$) exhibit a low threshold voltage and low viscosity.

Compounds in which Ra and/or Rb is an optically active group are particularly useful as chiral dopant. Besides, when Rb is a halogen atom, halogen substituted alkyl group, or halogen substituted alkoxy group, the compounds exhibit a high $\Delta\varepsilon$ and when it is cyano group, the compounds exhibit a higher $\Delta\varepsilon$.

Compounds in which any one or more of $Z_1$ to $Z_3$ are difluoromethylenoxy group ($-CF_2O-$) or oxydifluoromethylene group ($-OCF_2-$) exhibit a comparatively high $\Delta\varepsilon$ and low viscosity and the compounds in which the substituents are 1,2-difluorovinylene (-CF=CF-) exhibit a considerably low viscosity.

Further, the compounds of the present invention in which an atom therein is replaced by its isotope can be said to be preferable since the compounds exhibit similar characteristics.

Based on these facts, novel liquid crystalline compounds having desired physical properties can be obtained by selecting proper rings, substituents, and bonding groups.

Heretofore, liquid crystalline compounds having pyridine oxide ring in the molecular skeleton have little been known and only one compound is merely reported by M. P. Burrow (Liq. Cryst., 3, 1643 (1988)).

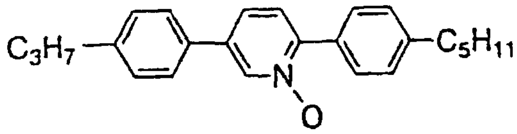

Liquid crystal compositions of the present invention are described below. Liquid crystal compositions of the present invention preferably comprise at least one compound expressed by the general formula (1) in the ratio of 0.1 to 99.9 % by weight to develop excellent characteristics and the ratio is more desirably 1 to 60 % by weight.

In more detail, the liquid crystal compositions provided according to the present invention are completed by mixing the compounds selected from the group consisting of the compounds expressed by at least one of the general formulas (2) to (12) depending on the object of the liquid crystal compositions in addition to a first component comprising at least one compound expressed by the general formula (1).

As preferable examples of the compounds used in the liquid crystal compositions of the present invention and expressed by one of the general formulas (2) to (4), the compounds expressed by one of the following general formulas can be mentioned:

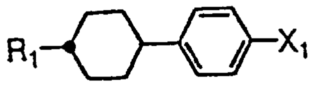

(2-1)

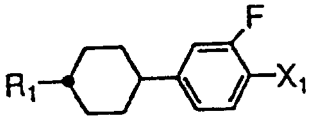

(2-2)

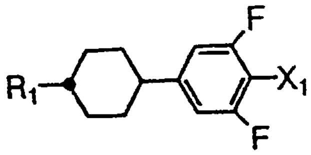

(2-3)

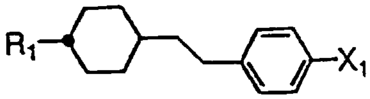

(2-4)

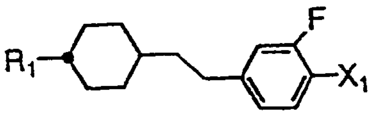

(2-5)

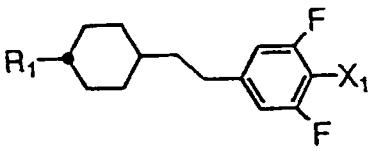

(2-6)

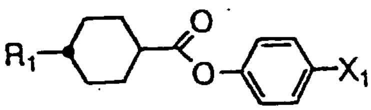

(2-7)

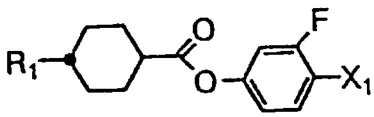

(2-8)

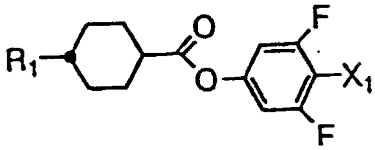

(2-9)

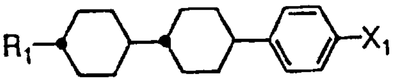
(3-1)

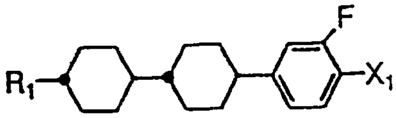
(3-2)

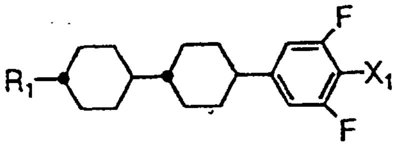
(3-3)

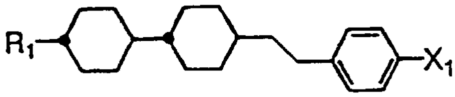
(3-4)

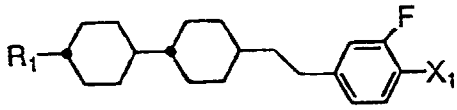
(3-5)

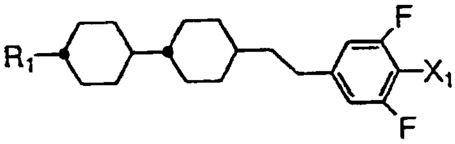
(3-6)

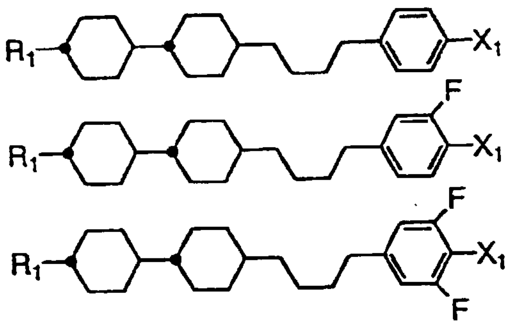
(3-7)

(3-8)

(3-9)

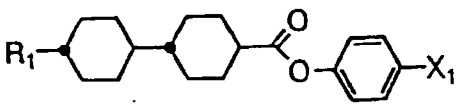
(3-10)

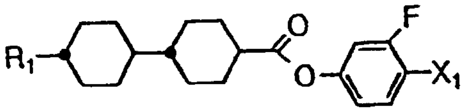
(3-11)

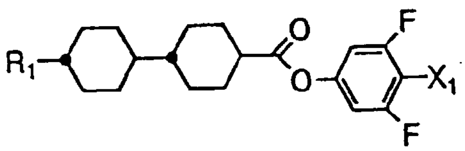
(3-12)

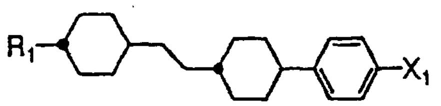
(3-13)

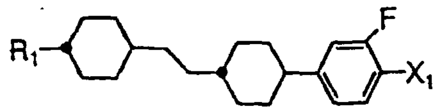
(3-14)

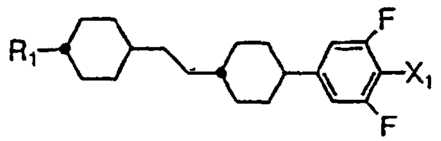
(3-15)

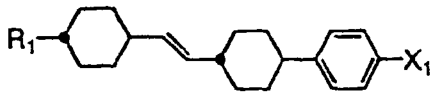
(3-16)

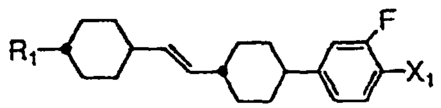
(3-17)

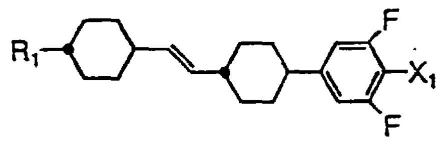
(3-18)

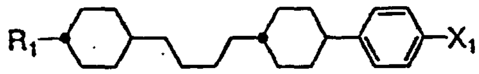
(3-19)

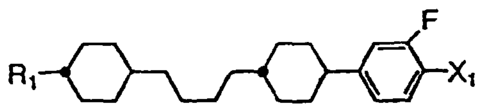
(3-20)

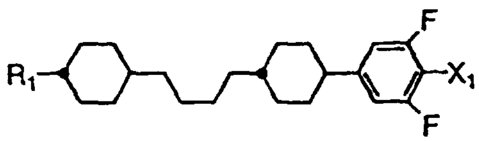
(3-21)

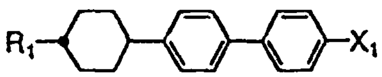
(3-22)

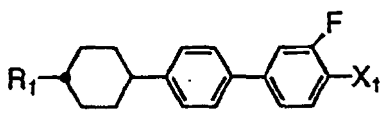
(3-23)

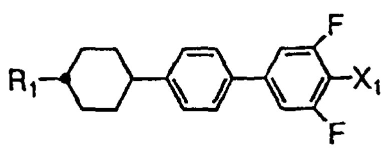
(3-24)

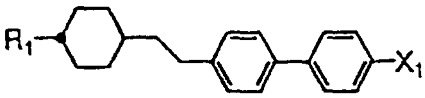

(3-25)

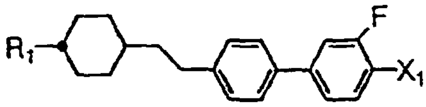

(3-26)

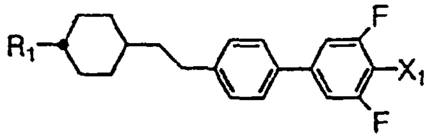

(3-27)

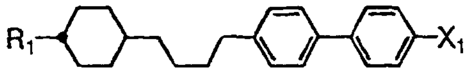

(3-28)

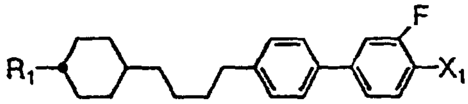

(3-29)

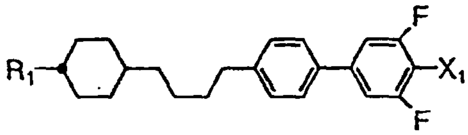

(3-30)

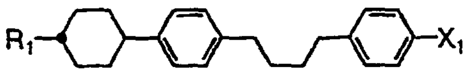

(3-31)

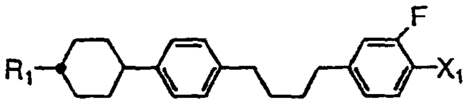

(3-32)

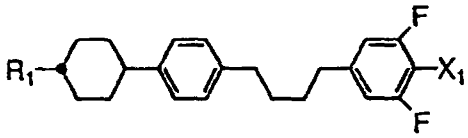

(3-33)

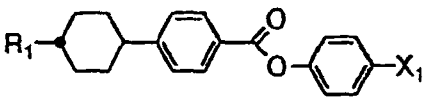

(3-34)

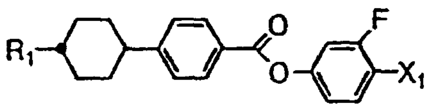

(3-35)

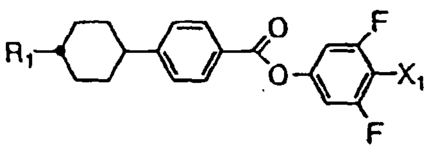

(3-36)

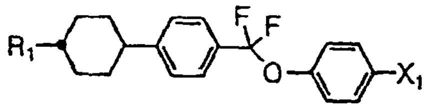

(3-37)

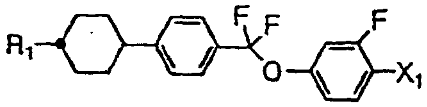

(3-38)

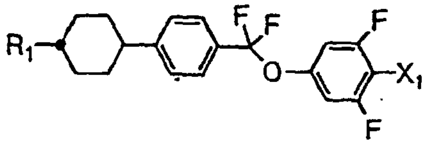

(3-39)

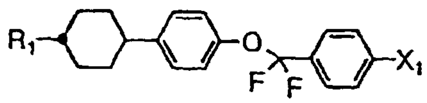

(3-40)

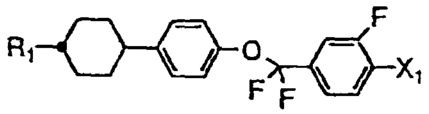

(3-41)

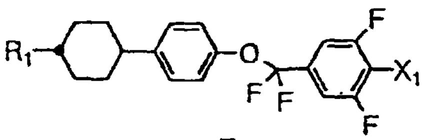

(3-42)

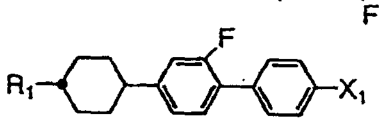

(3-43)

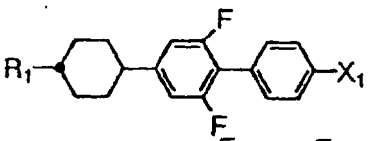

(3-44)

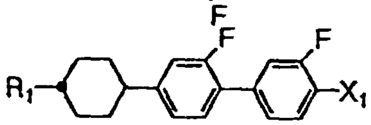

(3-45)

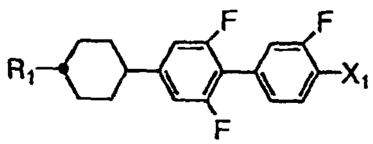

(3-46)

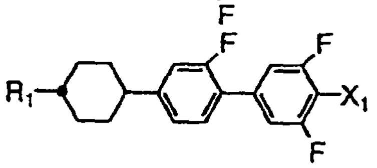

(3-47)

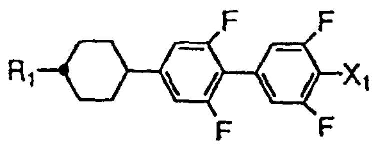

(3-48)

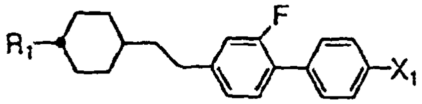

(3-49)

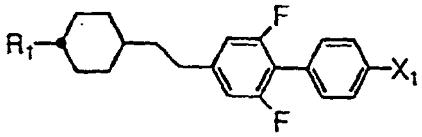

(3-50)

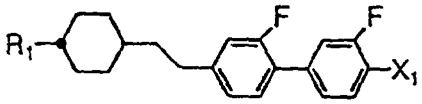

(3-51)

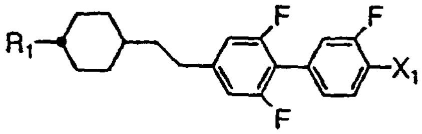

(3-52)

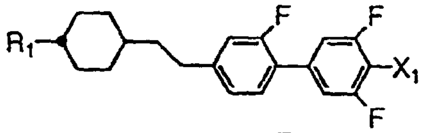

(3-53)

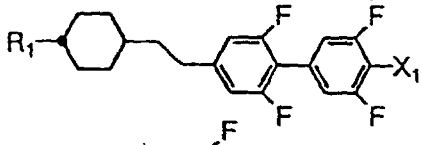

(3-54)

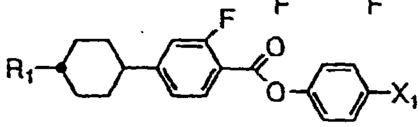

(3-55)

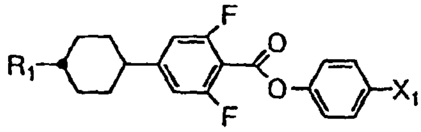

(3-56)

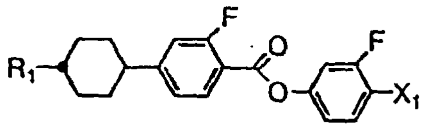

(3-57)

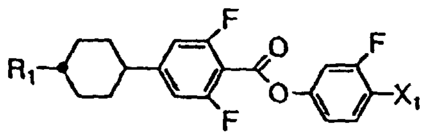

(3-58)

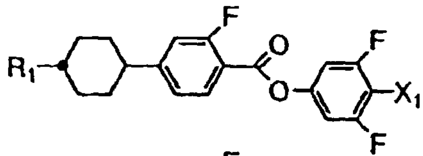

(3-59)

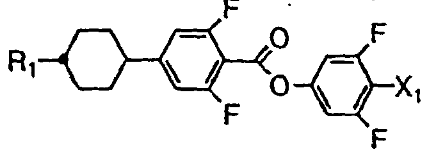

(3-60)

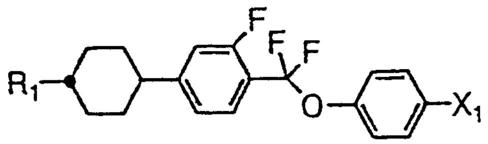

(3-61)

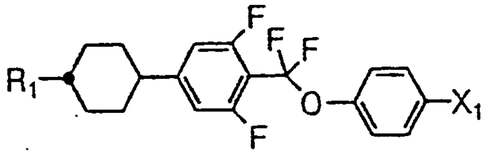

(3-62)

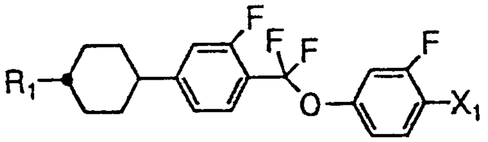

(3-63)

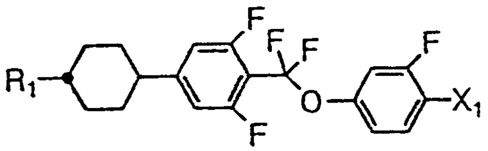

(3-64)

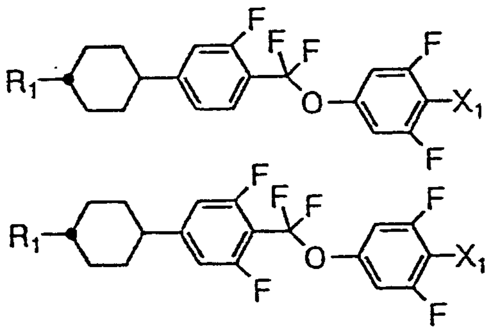

(3-65)

(3-66)

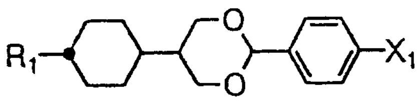

(3-67)

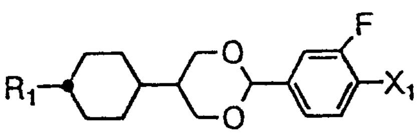

(3-68)

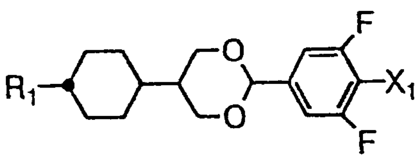

(3-69)

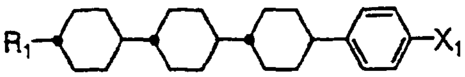
(4-1)

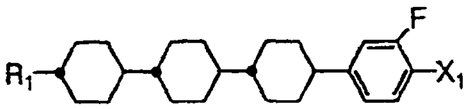
(4-2)

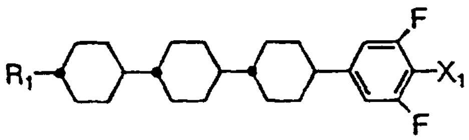
(4-3)

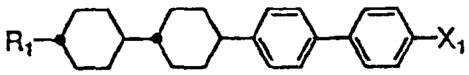
(4-4)

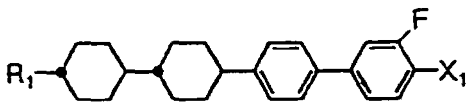
(4-5)

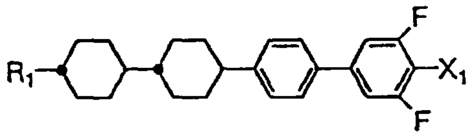
(4-6)

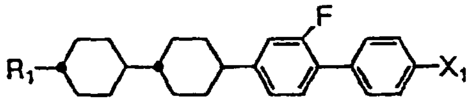
(4-7)

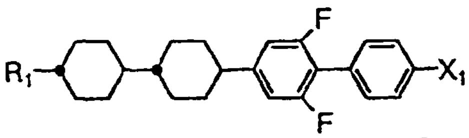
(4-8)

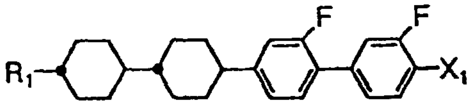
(4-9)

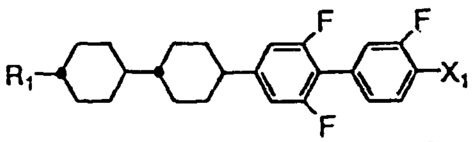
(4-10)

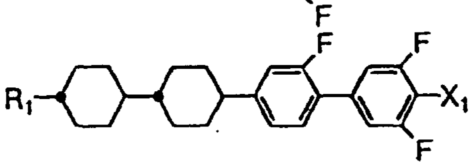
(4-11)

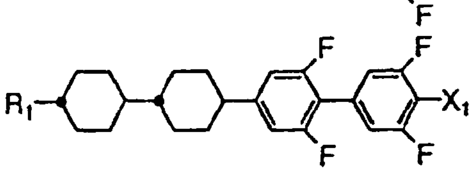
(4-12)

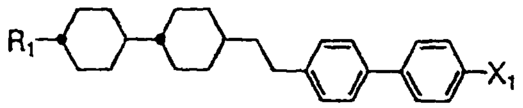

(4-13)

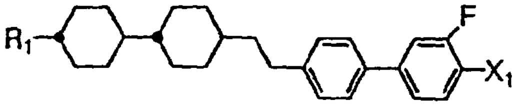

(4-14)

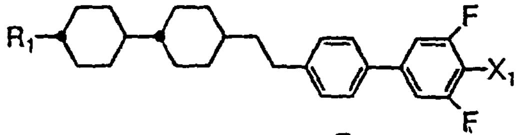

(4-15)

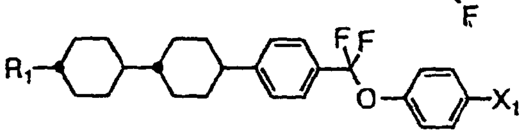

(4-16)

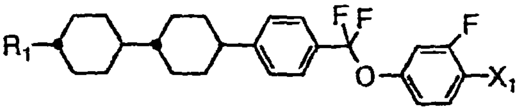

(4-17)

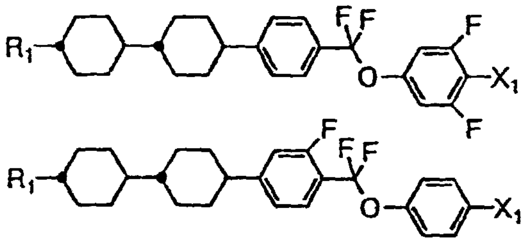

(4-18)

(4-19)

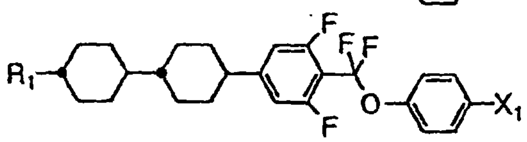

(4-20)

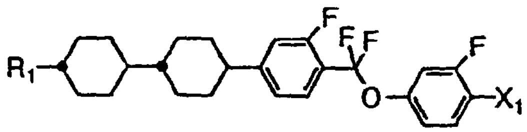

(4-21)

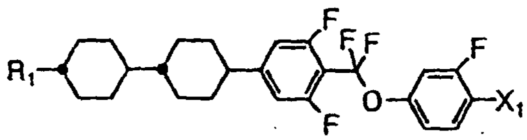

(4-22)

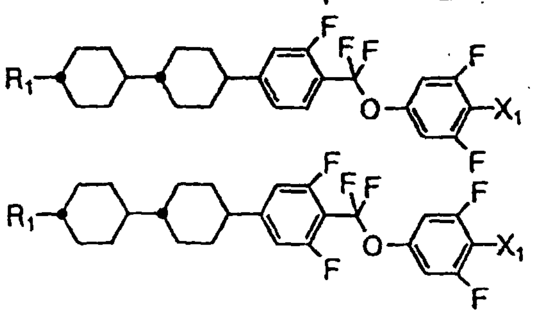

(4-23)

(4-24)

wherein $R_1$ and $X_1$ have the same meaning as described above.

Compounds expressed by one of the general formulas (2) to (4) have a positive dielectric anisotropy value, are remarkably excellent in thermal stability and chemical stability, and are very useful when liquid crystal compositions for TFT of which a high reliability particularly such as a high voltage holding ratio (or large specific resistivity) is required.

When liquid crystal compositions for TFT are produced, the compound expressed by one of the general formulas (2) to (4) may be used in the range of 0.1 to 99.9 % by weight based on the total amount of liquid crystal composition, and the amount is preferably 10 to 97 % by weight and more desirably 40 to 95 % by weight. Besides, the compositions may contain the compound expressed by one of the general formulas (10) to (12) for the purpose of adjusting viscosity.

Also, when liquid crystal compositions for STN or TN are produced, the compound expressed by one of the general formulas (2) to (4) may be used, but the amount of the compound to be used is preferably less than 50 % by weight.

As preferable examples of the compounds used in the liquid crystal compositions of the present invention and expressed by the general formula (5) or (6), the compounds expressed by one of the following general formulas can be mentioned:

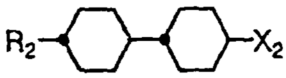

(5-1)

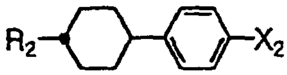

(5-2)

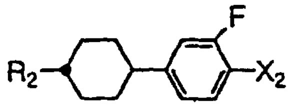

(5-3)

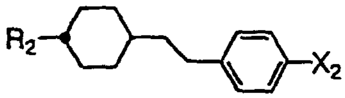

(5-4)

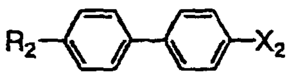

(5-5)

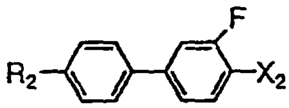

(5-6)

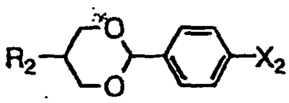

(5-7)

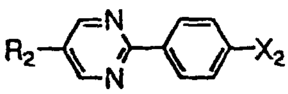

(5-8)

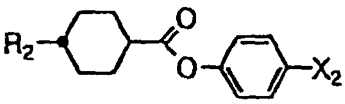

(5-9)

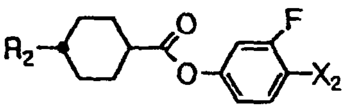

(5-10)

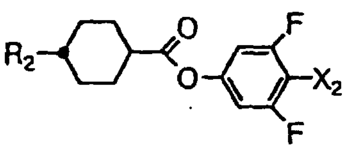

(5-11)

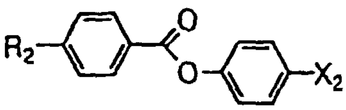

(5-12)

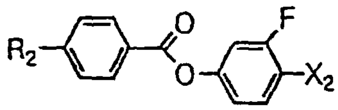 (5-13)

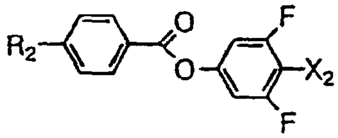 (5-14)

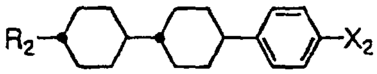 (5-15)

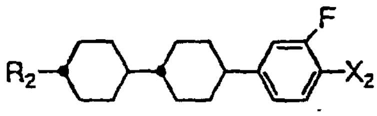 (5-16)

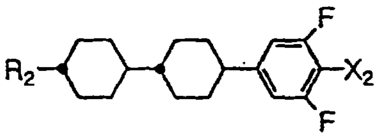 (5-17)

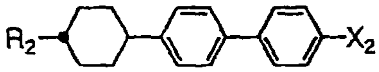 (5-18)

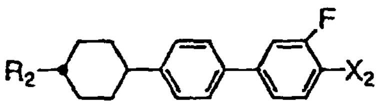 (5-19)

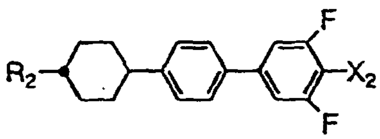 (5-20)

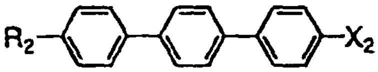 (5-21)

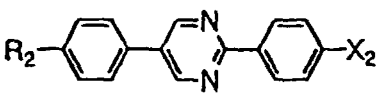 (5-22)

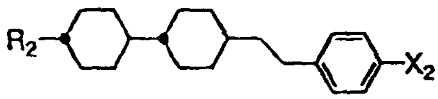 (5-23)

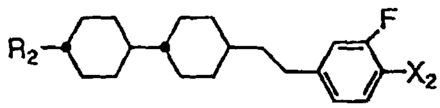 (5-24)

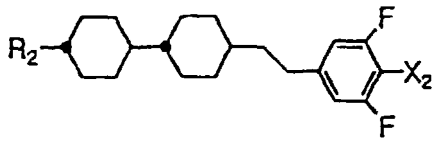

(5-25)

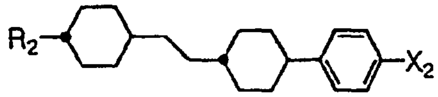

(5-26)

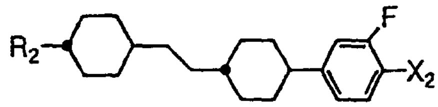

(5-27)

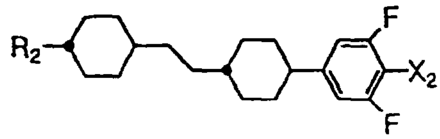

(5-28)

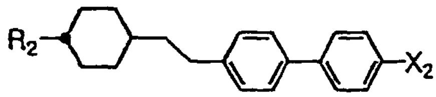

(5-29)

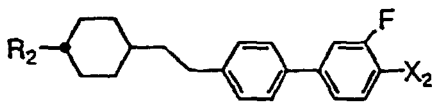

(5-30)

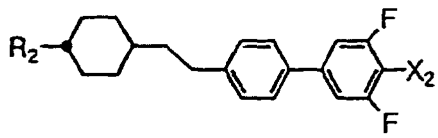

(5-31)

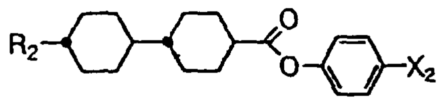

(5-32)

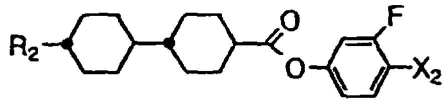

(5-33)

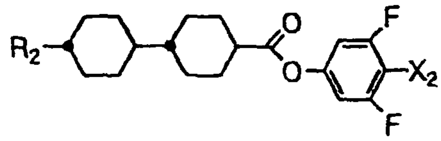

(5-34)

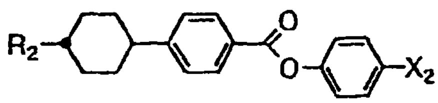

(5-35)

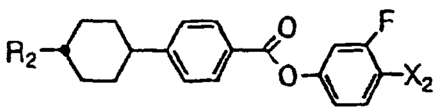

(5-36)

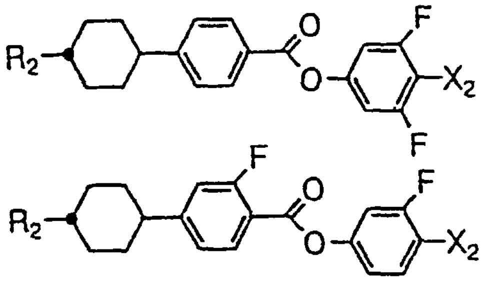

(5-37)

(5-38)

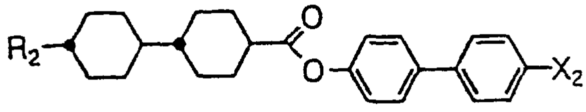

(5-39)

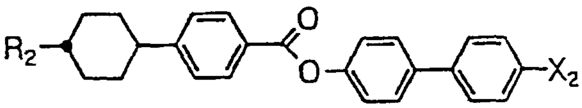

(5-40)

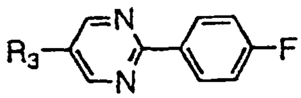

(6-1)

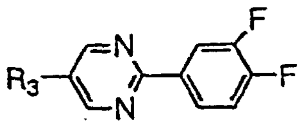

(6-2)

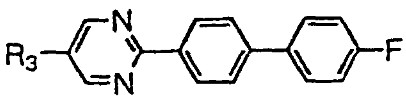

(6-3)

wherein $R_2$, $R_3$, and $X_2$ have the same meaning as described above.

Compounds expressed by the general formula (5) or (6) have a positive and large dielectric anisotropy value and are used particularly for the purpose of lowering threshold voltage of liquid crystal compositions. Also, they are used for the purpose of adjusting optical anisotropy value and widening nematic range such as raising clearing point. Further, they are used even for the purpose of improving the steepness of voltage-transmittance characteristic of liquid crystal compositions for STN or TN.

Compounds expressed by the general formula (5) or (6) are particularly useful when liquid crystal compositions for STN or TN are produced.

When the amount of the compound expressed by the general formula (5) or (6) in liquid crystal compositions is increased, threshold voltage of the liquid crystal compositions lowers but viscosity raises. Accordingly, it is advantage to use the compound in a large amount since the compositions can be driven at a low voltage so far as the viscosity of the liquid crystal compositions satisfies a required value. When liquid crystal compositions for STN or TN are produced, the compound expressed by the general formula (5) or (6) may be used in the range of 0.1 to 99.9 % by weight based on the total amount of liquid crystal composition, but the amount is preferably 10 to 97 % by weight and more desirably 40 to 95 % by weight.

As preferable examples of the compounds used in the liquid crystal compositions of the present invention and expressed by one of the general formulas (7) to (9), the compounds expressed by one of the following general formulas can be mentioned:

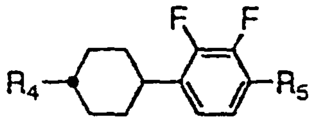

(7-1)

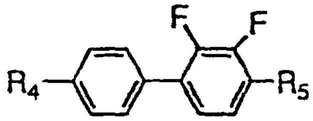

(7-2)

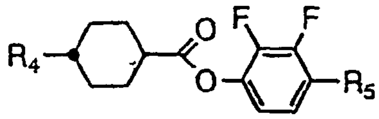

(7-3)

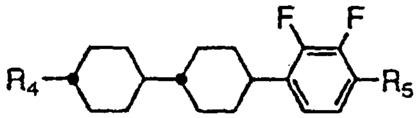

(8-1)

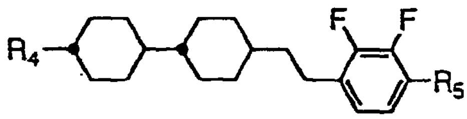

(8-2)

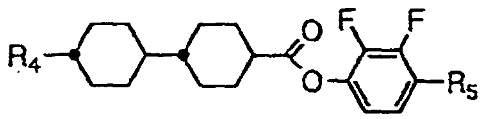

(8-3)

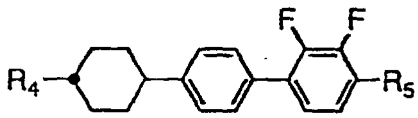

(8-4)

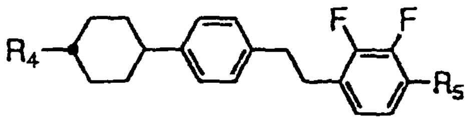

(8-5)

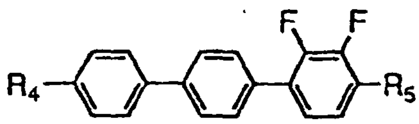

(9-1)

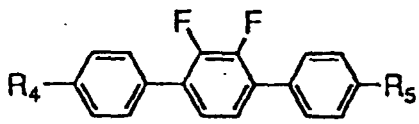

(9-2)

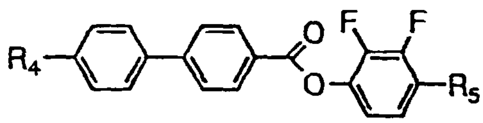

(9-3)

wherein $R_4$ and $R_5$ have the same meaning as described above.

Compounds expressed by one of the general formulas (7) to (9) have a negative dielectric anisotropy value. Since the compounds expressed by the general formula (7) are two-ring compounds, they are used principally for the purpose of adjusting threshold voltage, adjusting viscosity, or adjusting optical anisotropy value. Compounds expressed

by the general formula (8) are used for the purpose of widening nematic range such as raising clearing point or the purpose of adjusting optical anisotropy value. Compounds expressed by the general formula (9) are used for the purpose of lowering threshold voltage and for the purpose of increasing optical anisotropy value in addition to for the purpose of widening nematic range.

Compounds expressed by one of the general formulas (7) to (9) are principally used for liquid crystal compositions having a negative dielectric anisotropy value. When the amount of the compound expressed by one of the general formulas (7) to (9) in liquid crystal compositions is increased, threshold voltage of liquid crystal compositions lowers and viscosity raises. Accordingly, it is desirable to use the compound in a small amount so far as the threshold voltage of the liquid crystal compositions satisfies a required value. However, since the absolute value of dielectric anisotropy of the compounds is lower than 5, when the amount becomes less than 40 % by weight, it sometimes becomes impossible to drive at a low voltage. When liquid crystal compositions for TFT having a negative dielectric anisotropy are produced, the compound expressed by one of the general formulas (7) to (9) may be used in the range of more than 40 % by weight based on the total amount of liquid crystal composition, but the amount is preferably 50 to 95 % by weight.

Further, there is a case in which the compound expressed by one of the general formulas (7) to (9) is mixed in the compositions having a positive dielectric anisotropy. In this case, the amount of the compound expressed by one of the general formulas (7) to (9) is preferably less than 30 % by weight based on the total amount of liquid crystal composition.

As preferable examples of the compounds used in the liquid crystal compositions of the present invention and expressed by one of the general formulas (10) to (12), the compounds expressed by one of the following general formulas can be mentioned:

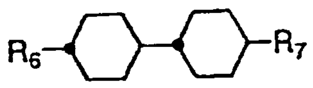

(10-1)

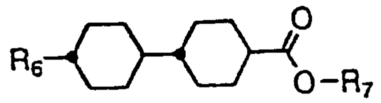

(10-2)

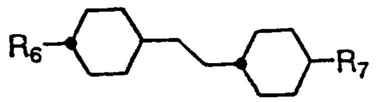

(10-3)

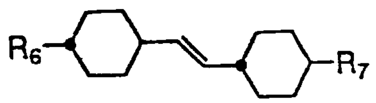

(10-4)

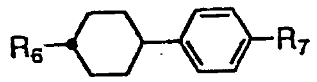

(10-5)

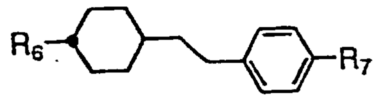

(10-6)

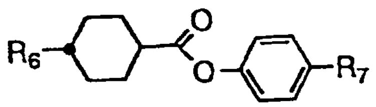

(10-7)

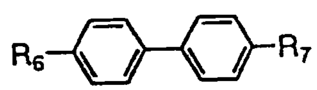

(10-8)

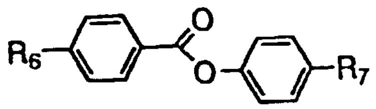

(10-9)

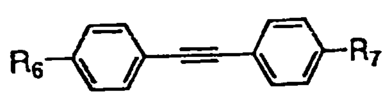

(10-10)

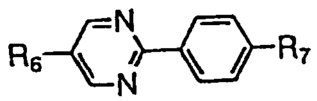

(10-11)

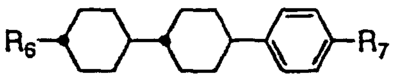

(11-1)

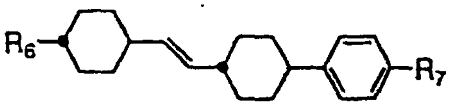

(11-2)

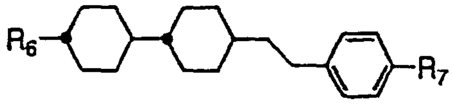

(11-3)

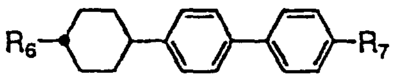

(11-4)

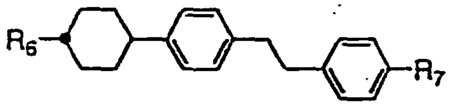

(11-5)

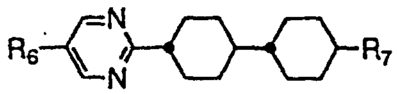

(11-6)

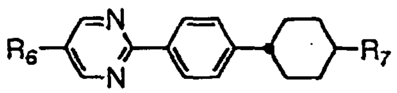

(11-7)

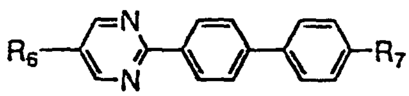

(11-8)

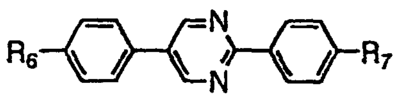

(11-9)

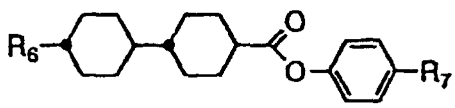

(11-10)

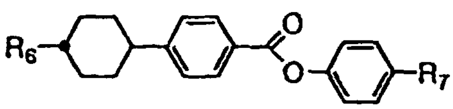

(11-11)

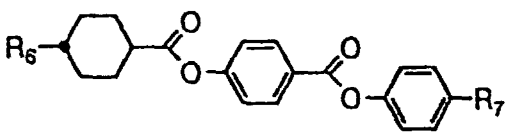

(11-12)

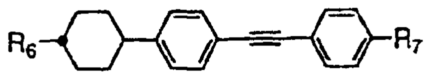

(11-13)

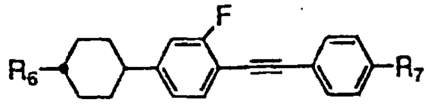

(11-14)

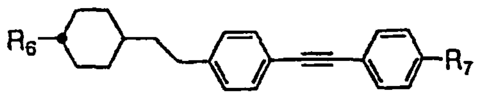

(11-15)

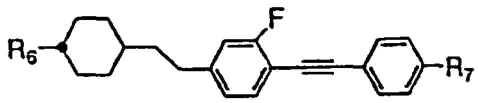

(11-16)

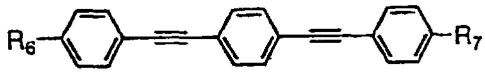

(11-17)

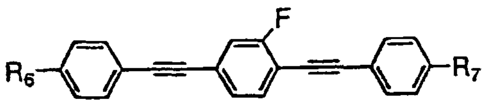

(11-18)

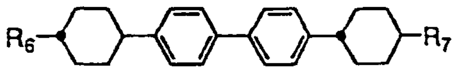

(12-1)

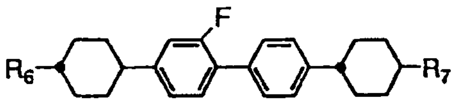

(12-2)

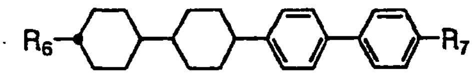

(12-3)

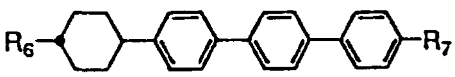

(12-4)

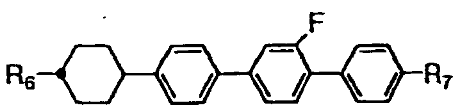

(12-5)

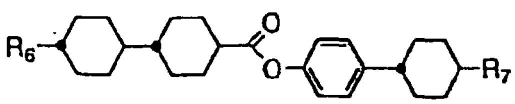

(12-6)

wherein $R_6$ and $R_7$ have the same meaning as described above.

Absolute value of dielectric anisotropy of the compounds expressed by one of the general formulas (10) to (12) is small and close to zero. Compounds expressed by the general formula (10) are used principally for the purpose of adjusting viscosity and adjusting optical anisotropy value. Compounds expressed by the general formula (11) or (12)

are used for the purpose of widening nematic range such as raising clearing point or for the purpose of adjusting optical anisotropy value.

When the amount of the compound expressed by one of the general formulas (10) to (12) in liquid crystal compositions is increased, threshold voltage of the liquid crystal compositions raises and viscosity reduces. Accordingly, it is desirable to use the compound in a large amount so far as the threshold voltage of the liquid crystal compositions satisfies a required value. When liquid crystal compositions for TFT are produced, the compound expressed by one of the general formulas (10) to (12) may be used in the range of less than 40 % by weight based on the total amount of liquid crystal composition, and the amount is more desirably less than 35 % by weight. Besides, when liquid crystal compositions for STN or TN are produced, the amount of the compound expressed by one of the general formulas (10) to (12) is preferably less than 70 % by weight and more desirably less than 60 % by weight.

Further, with the exception of such specific cases as liquid crystal compositions for OCB (Optically Compensated Birefringence) mode or liquid crystal compositions for VA mode, an optically active compound is added to the liquid crystal compositions of the present invention for the purpose of inducing helical structure of liquid crystal compositions to adjust required twist angle and to prevent reverse twist. For such purposes, while any known optically active compounds can be used, the following optically active compounds can be mentioned as preferable examples.

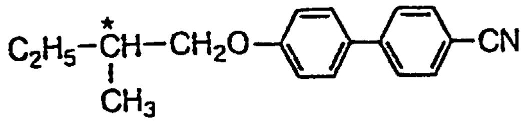

Symbol: C15

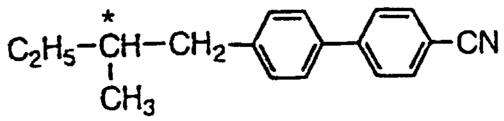

Symbol: CB15

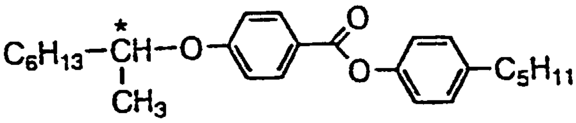

Symbol: CM21

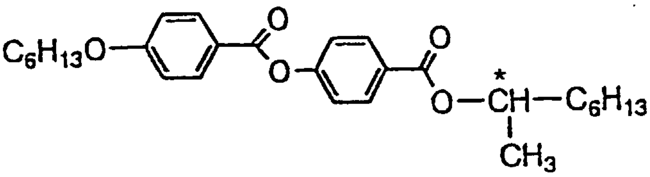

Symbol: CM33

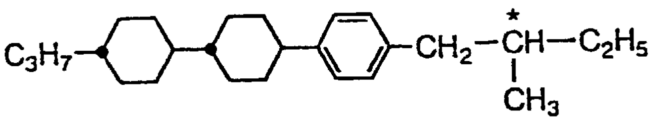

Symbol: CM43L

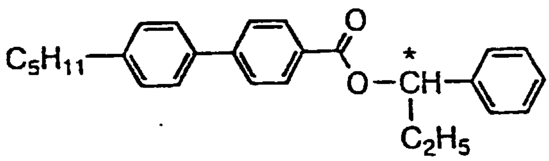

Symbol: CM45

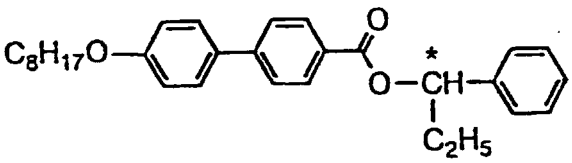

Symbol: CM47

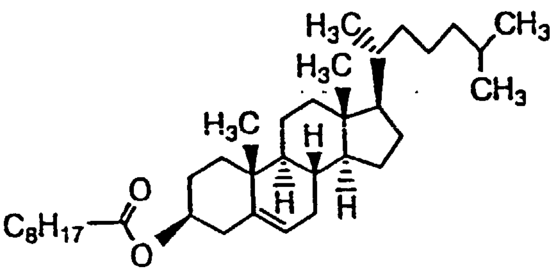

Symbol: CN

Usually, these optically active compounds are added to the liquid crystal compositions of the present invention to adjust the pitch of the twist. Pitch of the twist is preferably adjusted in the range of 40 to 200 µm in the case of liquid crystal compositions for TFT or TN, and preferably adjusted in the range of 6 to 20 µm in the case of liquid crystal compositions for STN. In the case of liquid crystal compositions for bistable TN mode, it is preferably adjusted in the range of 1.5 to 4 µm. Besides, two or more kind of optically active compounds may be added for the purpose of adjusting the dependency of the pitch on temperature.

Liquid crystal compositions of the present invention can be produced by conventional methods. Generally, a method in which various components are dissolved in one another at a high temperature is adopted.

Further, the liquid crystal compositions of the present invention can be used as ones for guest-host (GH) mode by adding a dichroic dye such as merocyanine type, styryl type, azo type, azomethine type, azoxy type, quinophthalone type, anthraquinone type, and tetrazine type thereto. Alternatively, the liquid crystal compositions can be used as NCAP which is prepared by the microencapsulation of a nematic liquid crystal, or as liquid crystal compositions for polymer dispersed liquid crystal display devices (PDLCD) represented by polymer net work liquid crystal display devices (PNLCD) prepared by forming a polymer of three-dimensional reticulated structure in a liquid crystal. Still further, the liquid crystal compositions of the present invention can be used as ones for electrically controlled birefringence (ECB) mode or dynamic scattering (DS) mode.

As examples of the liquid crystal compositions comprising the compound of the present invention, the following can be mentioned. Compounds in the following Composition Examples and Examples described below are designated by symbolizing the groups and ring structures in the molecule according to the definitions shown below.

Table 1 Method for designating compounds by using symbols

$R-(A_1)-Z_1-\cdots\cdots-Z_n-(A_n)-X$

| 1) Left side terminal group R- | Symbol | 3) Bonding group $-Z_1-$, $-Z_n-$ | Symbol |
|---|---|---|---|
| $C_nH_{2n+1}-$ | n— | $-C_2H_4-$ | 2 |
| $C_nH_{2n+1}O-$ | nO— | $-C_4H_8-$ | 4 |
| $C_nH_{2n+1}OC_mH_{2m}-$ | nOm— | $-COO-$ | E |
| $CH_2=CH-$ | V— | $-C\equiv C-$ | T |
| $CH_2=CHC_nH_{2n}-$ | Vn— | $-CH=CH-$ | V |
| $C_nH_{2n+1}CH=CHC_mH_{2m}-$ | nVm— | $-CF_2O-$ | CF2O |

| 2) Ring structure $-(A_1)-$, $-(A_n)-$ | Symbol | 4) Right side terminal group -X | Symbol |
|---|---|---|---|
| 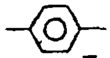 | B | $-F$ | — F |
| 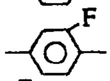 | B(F) | $-Cl$ | —CL |
| 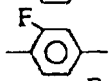 | B(2F) | $-CN$ | —C |
| 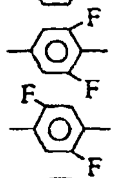 | B(F,F) | $-CF_3$ | —CF3 |
| | B(2F,3F) | $-OCF_3$ | —OCF3 |
| 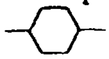 | H | $-OCF_2H$ | — OCF2H |
| 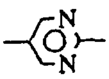 | Py | $-C_nH_{2n+1}$ | — n |
| 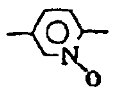 | Po | $-OC_nH_{2n+1}$ | —On |
| 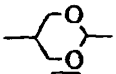 | G | $-COOCH_3$ | — EMe |
| 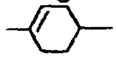 | Ch | $-C_nH_{2n}CH=CH_2$ | — n V |
| | | $-C_mH_{2m}CH=CHC_nH_{2n+1}$ | — mVn |
| | | $-CH=CF_2$ | — VFF |
| | | $-C_nH_{2n}CH=CF_2$ | — nVFF |
| | | $-CH=CHC_nH_{2n}F$ | — VnF |
| | | $-CH=CH_2$ | — V |
| | | $-C\equiv C-CN$ | — TC |

5) Examples of designation

Example 1 3-H2B(F,F)B(F)-F

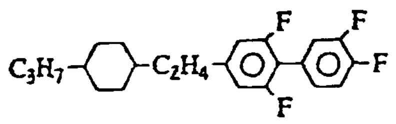

Example 3 1V2-BEB(F,F)-C

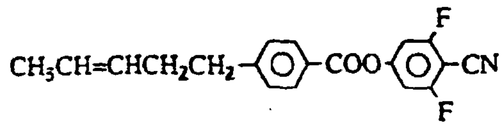

Example 2 3-HB(F)TB-2

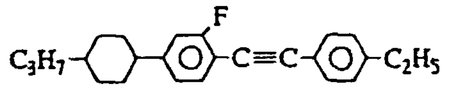

Further, for instance, when the hydrogen atoms of trans-1,4-cyclohexylene in the following partial structure were replaced by heavy hydrogen atoms (deuteriums) at positions $Q_1$, $Q_2$, $Q_3$, and $Q_4$, they are designated by symbol: H[1D 2D, 3D, 4D], and when replaced at positions $Q_5$, $Q_6$, $Q_7$, and $Q_8$, they are designated by symbol: H[5D, 6D, 7D, 8D]. In other words, the positions where the heavy hydrogen atoms substituted are indicated by the numeral in the bracket [ ].

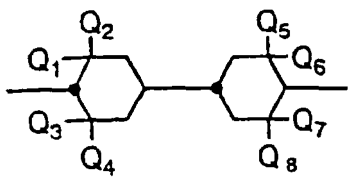

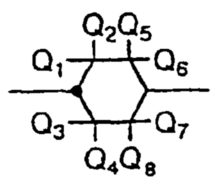

In the Composition Examples and Examples, "%" means % by weight unless other wise specified. When cis-trans isomers exist with a compound, the type of isomerism of the compound is trans-form. Besides, the viscosity (η) was determined at 20°C, and optical anisotropy (Δn), dielectric anisotropy (Δε), threshold voltage (Vth), and twist pitch (P) were determined each at 25°C.

Composition Example 1

| | |
|---|---|
| 8-PoB-O6 | 10.0% |
| 3-HEB-O4 | 25.0% |
| 4-HEB-O2 | 19.0% |

(continued)

| | |
|---|---|
| 5-HEB-O1 | 19.0% |
| 3-HEB-O2 | 15.0% |
| 5-HEB-O2 | 12.0% |
| $T_{NI}$ = 69.9 (°C) | |
| η = 40.9 (mPa • s) | |
| Δn = 0.091 | |
| Δε = -1.7 | |

Composition Example 2

| | |
|---|---|
| 8-PoB-O6 | 5.0% |
| 3-HH-2 | 5.0% |
| 3-HH-4 | 6.0% |
| 3-HH-O1 | 4.0% |
| 3-HH-O3 | 5.0% |
| 5-HH-O1 | 4.0% |
| 3-HB(2F,3F)-O2 | 12.0% |
| 5-HB(2F,3F)-O2 | 11.0% |
| 3-HHB(2F,3F)-O2 | 14.0% |
| 5-HHB(2F,3F)-O2 | 10.0% |
| 3-HHB(2F,3F)-2 | 24.0% |
| $T_{NI}$ = 78.8 (°C) | |
| Δn = 0.082 | |
| Δε = -4.0 | |

Composition Example 3

| | |
|---|---|
| 8-PoB-O6 | 5.0% |
| 6-BPoB-O8 | 3.0% |
| 3-HH-5 | 5.0% |
| 3-HH-4 | 5.0% |
| 3-HH-O1 | 6.0% |
| 3-HH-O3 | 6.0% |
| 3-HB-O1 | 5.0% |

(continued)

| | |
|---|---|
| 3-HB-O2 | 5.0% |
| 3-HB(2F,3F)-O2 | 10.0% |
| 5-HB(2F,3F)-O2 | 5.0% |
| 3-HHB(2F,3F)-O2 | 12.0% |
| 5-HHB(2F,3F)-O2 | 10.0% |
| 3-HHB(2F,3F)-2 | 4.0% |
| 2-HHB(2F,3F)-1 | 4.0% |
| 3-HHEH-3 | 5.0% |
| 3-HHEH-5 | 5.0% |
| 4-HHEH-3 | 5.0% |
| $T_{NI}$ = 86.2 (°C) | |
| $\Delta n$ = 0.083 | |
| $\Delta\varepsilon$ = -3.2 | |

Composition Example 4

| | |
|---|---|
| 8-PoB-O6 | 10.0% |
| 6-BPoB-O8 | 5.0% |
| 3-HPoB(F)-F | 5.0% |
| 3-BB(2F,3F)-O2 | 12.0% |
| 3-BB(2F,3F)-O4 | 10.0% |
| 2-BB(2F,3F)B-3 | 25.0% |
| 3-BB(2F,3F)B-5 | 10.0% |
| 5-BB(2F,3F)B-5 | 10.0% |
| 5-BB(2F,3F)B-7 | 13.0% |

Composition Example 5

| | |
|---|---|
| 8-PoB-O6 | 10.0% |
| 6-BPoB-O8 | 10.0% |
| 3-HPoB(F)-F | 10.0% |
| 3-BB(2F,3F)-O2 | 5.0% |
| 5-BB-5 | 9.0% |
| 5-BB-O6 | 9.0% |
| 5-BB-O8 | 8.0% |

(continued)

| | |
|---|---|
| 1-BEB-5 | 6.0% |
| 3-BEB-5 | 4.0% |
| 3-HEB-O2 | 10.0% |
| 5-BBB(2F,3F)-7 | 9.0% |
| 3-H2BB(2F)-5 | 10.0% |

Composition Example 6

| | |
|---|---|
| 6-BPoB-O8 | 10.0% |
| 3-HPoB(F)-F | 5.0% |
| 3-HB-O1 | 15.0% |
| 3-HB-O2 | 6.0% |
| 3-HEB(2F,3F)-O2 | 9.0% |
| 4-HEB(2F,3F)-O2 | 5.0% |
| 5-HEB(2F,3F)-O2 | 4.0% |
| 2-BB2B-O2 | 6.0% |
| 3-BB2B-O2 | 6.0% |
| 5-BB2B-O1 | 6.0% |
| 1-B2BB(2F)-5 | 7.0% |
| 3-B2BB(2F)-5 | 7.0% |
| 5-B(F)BB-O2 | 7.0% |
| 3-BB(2F,3F)B-3 | 7.0% |

Composition Example 7

| | |
|---|---|
| 6-BPoB-O8 | 5.0% |
| 3-HB-O1 | 9.0% |
| 3-HB-O2 | 9.0% |
| 3-HB-O4 | 9.0% |
| 2-BTB-O1 | 5.0% |
| 1-BTB-O2 | 5.0% |
| 3-BTB(2F,3F)-O2 | 13.0% |
| 5-BTB(2F,3F)-O2 | 13.0% |
| 3-B(2F,3F)TB(2F,3F)-O4 | 4.0% |

(continued)

| | |
|---|---|
| 5-B(2F,3F)TB(2F,3F)-O4 | 4.0% |
| 3-HBTB-O1 | 5.0% |
| 3-HBTB-O2 | 5.0% |
| 3-HBTB-O3 | 5.0% |
| 3-HHB(2F,3F)-O2 | 6.0% |
| 5-BPr(F)-O2 | 3.0% |
| $T_{NI}$ = 83.4 (°C) | |
| $\Delta n$ = 0.221 | |

Composition Example 8

| | |
|---|---|
| 8-PoB-O6 | 10.0% |
| 3-HB-O2 | 10.0% |
| 5-HB-3 | 8.0% |
| 5-BB(2F,3F)-O2 | 10.0% |
| 3-HB(2F,3F)-O2 | 5.0% |
| 5-HB(2F,3F)-O2 | 8.0% |
| 3-HHB(2F,3F)-O2 | 12.0% |
| 5-HHB(2F,3F)-O2 | 4.0% |
| 5-HHB(2F,3F)-1O1 | 4.0% |
| 2-HHB(2F,3F)-1 | 5.0% |
| 3-HBB-2 | 6.0% |
| 3-BB(2F,3F)B-3 | 8.0% |
| 5-B2BB(2F,3F)B-O2 | 10.0% |
| $T_{NI}$ = 69.8 (°C) | |
| $\Delta n$ = 0.131 | |
| $\Delta\varepsilon$ = -4.0 | |

Composition Example 9

| | |
|---|---|
| 8-PoB-O6 | 10.0% |
| 3-HE-O2 | 20.0% |
| 1O1-HH-3 | 3.0% |
| 1O1-HH-5 | 5.0% |

(continued)

| | |
|---|---|
| 3-HH-EMe | 12.0% |
| 4-HEB-O1 | 9.0% |
| 4-HEB-O2 | 7.0% |
| 5-HEB-O1 | 8.0% |
| 3-HHB-1 | 6.0% |
| 3-HHB-3 | 6.0% |
| 6-HEB(2CN,3CN)-O4 | 3.0% |
| 4-HEB(2CN,3CN)-O5 | 3.0% |
| 5-HEB(2CN,3CN)-O5 | 2.0% |
| 2-HBEB(2CN,3CN)-O2 | 2.0% |
| 4-HBEB(2CN,3CN)-O4 | 4.0% |
| $T_{NI}$ = 67.8 (°C) | |
| $\Delta n$ = 0.087 | |
| $\eta$ = 50.2 (mPa • s) | |
| $\Delta\varepsilon$ = -5.4 | |

Composition Example 10

| | |
|---|---|
| 8-PoB-O6 | 5.0% |
| 1V2-BEB(F,F)-C | 5.0% |
| 3-HB-C | 20.0% |
| V2-HB-C | 6.0% |
| 1-BTB-3 | 5.0% |
| 2-BTB-1 | 10.0% |
| 1O1-HH-3 | 3.0% |
| 3-HH-4 | 11.0% |
| 3-HHB-1 | 9.0% |
| 3-H2BTB-2 | 4.0% |
| 3-H2BTB-3 | 4.0% |
| 3-H2BTB-4 | 4.0% |
| 3-HB(F)TB-2 | 6.0% |
| 3-HB(F)TB-3 | 5.0% |
| 3-HHB-C | 3.0% |
| $T_{NI}$ = 85.4 (°C) | |
| $\eta$ = 22.6 (mPa • s) | |
| $\Delta n$ = 0.161 | |
| $\Delta\varepsilon$ = 7.0 | |

(continued)

| | |
|---|---|
| $V_{th}$ = 2.11 (V) | |

Pitch of the composition prepared by mixing 100 parts by weight of the composition shown in Composition Example 10 above with 0.8 part by weight of optically active compound CM-33 was 11.3 μm.

Composition Example 11

| | |
|---|---|
| 8-PoB-O6 | 3.0% |
| 6-BPoB-O8 | 3.0% |
| 5-PyB-F | 4.0% |
| 3-PyB(F)-F | 4.0% |
| 2-BB-C | 5.0% |
| 4-BB-C | 4.0% |
| 5-BB-C | 5.0% |
| 2-PyB-2 | 2.0% |
| 3-PyB-2 | 2.0% |
| 4-PyB-2 | 2.0% |
| 6-PyB-O5 | 3.0% |
| 6-PyB-O6 | 3.0% |
| 6-PyB-O8 | 3.0% |
| 3-PyBB-F | 6.0% |
| 4-PyBB-F | 6.0% |
| 5-PyBB-F | 6.0% |
| 3-HHB-1 | 3.0% |
| 3-HHB-3 | 8.0% |
| 2-H2BTB-2 | 4.0% |
| 2-H2BTB-3 | 4.0% |
| 2-H2BTB-4 | 5.0% |
| 3-H2BTB-2 | 5.0% |
| 3-H2BTB-3 | 5.0% |
| 3-H2BTB-4 | 5.0% |
| $T_{NI}$ = 92.3 (°C) | |
| η = 43.8 (mPa • s) | |
| Δn = 0.203 | |
| Δε = 6.2 | |
| $V_{th}$ = 2.30 (V) | |

Composition Example 12

| | |
|---|---|
| 3-HPoB(F)-F | 10.0% |
| 2O1-BEB(F)-C | 5.0% |
| 3O1-BEB(F)-C | 12.0% |
| 5O1-BEB(F)-C | 4.0% |
| 1V2-BEB(F,F)-C | 10.0% |
| 3-HEB-O4 | 4.0% |
| 3-HH-EMe | 6.0% |
| 3-HB-O2 | 13.0% |
| 7-HEB-F | 2.0% |
| 3-HHEB-F | 2.0% |
| 5-HHEB-F | 2.0% |
| 3-HBEB-F | 4.0% |
| 2O1-HBEB(F)-C | 2.0% |
| 3-HB(F)EB(F)-C | 2.0% |
| 3-HBEB(F,F)-C | 2.0% |
| 3-HHB-F | 4.0% |
| 3-HHB-O1 | 4.0% |
| 3-HHB-3 | 4.0% |
| 3-HEBEB-F | 2.0% |
| 3-HEBEB-1 | 2.0% |
| 3-HHB(F)-C | 4.0% |

Composition Example 13

| | |
|---|---|
| 8-PoB-O6 | 5.0% |
| 5-BEB(F)-C | 5.0% |
| V-HB-C | 11.0% |
| 5-PyB-C | 6.0% |
| 4-BB-3 | 11.0% |
| 5-HH-V2V | 4.0% |
| 3-HH-2V | 10.0% |
| 5-HH-V | 2.0% |
| V-HHB-1 | 7.0% |

(continued)

| | |
|---|---|
| V2-HHB-1 | 15.0% |
| 3-HHB-1 | 9.0% |
| 1V2-HBB-2 | 10.0% |
| 3-HHEBH-3 | 5.0% |
| $T_{NI}$ = 90.0 (°C) | |
| η = 24.4 (mPa • s) | |
| Δn = 0.119 | |
| Δε = 4.8 | |
| $V_{th}$ = 2.36 (V) | |

Composition Example 14

| | |
|---|---|
| 8-PoB-O6 | 5.0% |
| 6-BPoB-O8 | 10.0% |
| V2-HB-TC | 10.0% |
| 3-HB-TC | 10.0% |
| 3-HB-C | 10.0% |
| 5-HB-C | 7.0% |
| 5-BB-C | 3.0% |
| 2-BTB-1 | 6.0% |
| 2-BTB-O1 | 5.0% |
| 3-HH-4 | 5.0% |
| 3-HHB-1 | 10.0% |
| 3-H2BTB-2 | 3.0% |
| 3-H2BTB-3 | 3.0% |
| 3-HB(F)TB-2 | 3.0% |
| 5-BTB(F)TB-3 | 10.0% |
| $T_{NI}$ = 100.1 (°C) | |
| η = 42.2 (mPa • s) | |
| Δn = 0.204 | |
| Δε = 6.2 | |
| $V_{th}$ = 2.19 (V) | |

Composition Example 15

| | |
|---|---|
| 8-PoB-O6 | 5.0% |
| 1V2-BEB(F,F)-C | 6.0% |
| 3-HB-C | 18.0% |
| 2-BTB-1 | 10.0% |
| 5-HH-VFF | 25.0% |
| 1-BHH-VFF | 8.0% |
| 1-BHH-2VFF | 11.0% |
| 3-H2BTB-2 | 5.0% |
| 3-H2BTB-3 | 4.0% |
| 3-H2BTB-4 | 4.0% |
| 3-HHB-1 | 4.0% |
| $T_{NI}$ = 79.4 (°C) | |
| η = 20.8 (mpa • s) | |
| Δn = 0.132 | |
| Δε = 6.3 | |
| $V_{th}$ = 2.10 (V) | |

Composition Example 16

| | |
|---|---|
| 8-PoB-O6 | 5.0% |
| 3-HB-CL | 10.0% |
| 5-HB-CL | 4.0% |
| 7-HB-CL | 4.0% |
| 1O1-HH-5 | 5.0% |
| 2-HBB(F)-F | 8.0% |
| 3-HBB(F)-F | 8.0% |
| 5-HBB(F)-F | 14.0% |
| 4-HHB-CL | 8.0% |
| 5-HHB-CL | 3.0% |
| 3-H2HB(F)-CL | 4.0% |
| 3-HBB(F,F)-F | 10.0% |
| 5-H2BB(F,F)-F | 9.0% |
| 3-HB(F)VB-2 | 4.0% |

(continued)

| | |
|---|---|
| 3-H2BTB-2 | 4.0% |
| $T_{NI}$ = 84.8 (°C) | |
| η = 27.2 (mPa • s) | |
| Δn = 0.126 | |
| Δε = 4.5 | |
| $V_{th}$ = 2.39 (V) | |

Composition Example 17

| | |
|---|---|
| 6-BPoB-O8 | 5.0% |
| 5-HB-F | 12.0% |
| 6-HB-F | 9.0% |
| 7-HB-F | 7.0% |
| 2-HHB-OCF3 | 7.0% |
| 3-HHB-OCF3 | 7.0% |
| 4-HHB-OCF3 | 7.0% |
| 3-HH2B-OCF3 | 4.0% |
| 5-HH2B-OCF3 | 4.0% |
| 3-HHB(F,F)-OCF3 | 5.0% |
| 3-HBB(F)-F | 10.0% |
| 5-HBB(F)-F | 10.0% |
| 3-HH2B(F)-F | 3.0% |
| 3-HB(F)BH-3 | 3.0% |
| 5-HBBH-3 | 3.0% |
| 3-HHB(F,F)-OCF2H | 4.0% |
| $T_{NI}$ = 86.2 (°C) | |
| η = 21.8 (mPa • s) | |
| Δn = 0.092 | |
| Δε = 4.1 | |
| $V_{th}$ = 2.43 (V) | |

Composition Example 18

| | |
|---|---|
| 8-PoB-O6 | 8.0% |
| 7-HB(F)-F | 5.0% |

(continued)

| | |
|---|---|
| 5-H2B(F)-F | 5.0% |
| 3-HB-O2 | 10.0% |
| 3-HH-4 | 2.0% |
| 3-HH[5D,6D,7D]-4 | 3.0% |
| 2-HHB(F)-F | 10.0% |
| 3-HHB(F)-F | 10.0% |
| 5-HH[5D,6D,7D]B(F)-F | 10.0% |
| 3-H2HB(F)-F | 5.0% |
| 2-HBB(F)-F | 3.0% |
| 3-HBB(F)-F | 3.0% |
| 5-HBB(F)-F | 6.0% |
| 2-H2BB(F)-F | 3.0% |
| 3-HHB-1 | 8.0% |
| 3-HHB-O1 | 5.0% |
| 3-HHB-3 | 4.0% |
| $T_{NI}$ = 85.2 (°C) | |
| $\eta$ = 29.4 (mPa • s) | |
| $\Delta n$ = 0.090 | |
| $\Delta\varepsilon$ = 2.7 | |
| $V_{th}$ = 2.72 (V) | |

Pitch of the composition prepared by mixing 100 parts by weight of the composition of Composition Example 18 described above with 0.3 part by weight of optically active compound CN was 77 µm.

Composition Example 19

| | |
|---|---|
| 8-PoB-O6 | 5.0% |
| 7-HB(F,F)-F | 5.0% |
| 3-H2HB(F,F)-F | 7.0% |
| 4-H2HB(F,F)-F | 6.0% |
| 3-HHB(F,F)-F | 10.0% |
| 4-HHB(F,F)-F | 5.0% |
| 3-HBB(F,F)-F | 10.0% |
| 3-HHEB(F,F)-F | 10.0% |
| 4-HHEB(F,F)-F | 3.0% |
| 5-HHEB(F,F)-F | 3.0% |
| 2-HBEB(F,F)-F | 3.0% |
| 3-HBEB(F,F)-F | 5.0% |

(continued)

| | |
|---|---|
| 5-HBEB(F,F)-F | 3.0% |
| 3-HDB(F,F)-F | 15.0% |
| 3-HBCF2OB-OCF3 | 4.0% |
| 3-HHBB(F,F)-F | 6.0% |
| $T_{NI}$ = 74.6 (°C) | |
| η = 40.6 (mPa • s) | |
| Δn = 0.086 | |
| Δε = 12.6 | |
| $V_{th}$ = 1.43 (V) | |

Composition Example 20

| | |
|---|---|
| 6-BPoB-O8 | 5.0% |
| 3-HPoB(F)-F | 5.0% |
| 5-H4HB(F,F)-F | 7.0% |
| 5-H4HB-OCF3 | 10.0% |
| 3-H4HB(F,F)-CF3 | 8.0% |
| 5-H4HB(F,F)-CF3 | 10.0% |
| 3-HB-CL | 6.0% |
| 5-HB-CL | 4.0% |
| 2-H2BB(F)-F | 5.0% |
| 3-H2BB(F)-F | 5.0% |
| 5-HVHB(F,F)-F | 5.0% |
| 3-HHB-OCF3 | 5.0% |
| 3-H2HB-OCF3 | 5.0% |
| V-HHB(F)-F | 5.0% |
| 3-HHB(F)-F | 5.0% |
| 5-HHEB-OCF3 | 2.0% |
| 3-HBEB(F,F)-F | 5.0% |
| 5-HH-V2F | 3.0% |

Composition Example 21

| | |
|---|---|
| 8-PoB-O6 | 10.0% |

(continued)

| | |
|---|---|
| 6-BPoB-O8 | 5.0% |
| 2-HHB(F)-F | 2.0% |
| 3-HHB(F)-F | 2.0% |
| 5-HHB(F)-F | 2.0% |
| 2-HBB(F)-F | 6.0% |
| 3-HBB(F)-F | 6.0% |
| 5-HBB(F)-F | 4.0% |
| 2-H2BB(F)-F | 9.0% |
| 3-HBB(F,F)-F | 25.0% |
| 5-HBB(F,F)-F | 19.0% |
| 1O1-HBBH-4 | 5.0% |
| 1O1-HBBH-5 | 5.0% |
| $T_{NI}$ = 92.7 (°C) | |
| η = 57.6 (mPa • s) | |
| Δn = 0.131 | |
| Δε = 5.8 | |
| $V_{th}$ = 2.05 (V) | |

Pitch of the composition prepared by mixing 100 parts by weight of the composition of Composition Example 21 described above with 0.2 part by weight of optically active compound CM-43L was 80 μm.

BEST MODE FOR CARRYING OUT THE INVENTION

Now, the present invention will be described in more detail with reference to Examples.

Example 1

Preparation of 5-octyl-2-(4-hexyloxyphenyl)pyridine-N-oxide

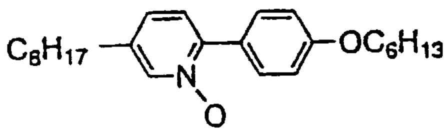

Mixture of 10 mmol of the 5-octyl-2-(4-hexyloxyphenyl)pyridine prepared according to the method of A. I. Pavlyuchenko et al., Khim. Geterotsikl. Soedin., 1389 (1985) and Adv. Liq. Cryst. Res. Appl., (Ed. L. Bata), 1007 (1980), 20 mmol of m-chloroperbenzoic acid, and 40 ml of chloroform was stirred at 5°C for 10 hours. Then, 5 mmol of m-chloroperbenzoic acid was added thereto and further stirred at the same temperature for 20 hours. The reaction solution was washed with 20 % aqueous sodium hydroxide solution and the solvent was removed under a reduced pressure to obtain a crude 5-octyl-2-(4-hexyloxyphenyl)pyridine-N-oxide.

After recrystallized from ethanol, it was subjected to silica gel column chromatography (eluent: ethyl acetate) and further recrystallized from heptane to obtain 11 mmol of the objective product.

This product exhibited a mesomorphism and its phase transition points were as follows:

| | |
|---|---|
| Melting point | : 108.5°C |
| SA phase - isotropic liquid phase | : 100.5°C |
| SC phase - SA phase | : 99°C |

Example 2 (Use Example 1)

Liquid crystal composition (A) comprising

| | |
|---|---|
| 4-ethoxyphenyl=4-propylcyclohexanecarboxylate | 17.2 % |
| 4-butoxyphenyl=4-propylcyclohexanecarboxylate | 27.6 % |
| 4-ethoxyphenyl=4-butylcyclohexanecarboxylate | 20.7 % |
| 4-methoxyphenyl=4-pentylcyclohexanecarboxylate | 20.7 % |
| 4-ethoxyphenyl=4-pentylcyclohexanecarboxylate | 13.8 % |

exhibited the following physical properties:

NI: 74.0°C $\Delta\varepsilon$: -1.3

Liquid crystal composition (B) comprising 90 % of the liquid crystal composition (A) and 10 % of the 5-octyl-2-(4-hexyloxyphenyl)pyridine-N-oxide obtained in Example 1 had the following physical property values. Values in the parentheses indicate the ones of 5-octyl-2-(4-hexyloxyphenyl)pyridine-N-oxide calculated by extrapolation.

NI: 69.4°C (28.0°C) $\Delta\varepsilon$: -1.78 (-4.93)

Example 3

Preparation of 6-fluoro-5-(4-hexylphenyl)-2-(4-octyloxyphenyl)pyridine-N-oxide

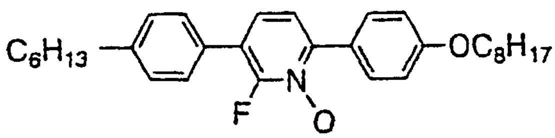

Mixture of 10 mmol of the 6-fluoro-5-(4-hexylphenyl)-2-(4-octyloxyphenyl)pyridine prepared according to the method of H. Schlosser (DE-OS 4,236,103), 10 ml of hydrogen peroxide solution, and 60 ml of acetic acid was stirred at 80°C for 4 hours. Then, 7 ml of hydrogen peroxide solution was added thereto and further stirred at the same temperature for 8 hours. The reaction solution was concentrated under a reduced pressure, the residue was dissolved in chloroform, the organic layer was washed with 20 % aqueous sodium hydroxide solution, and the solvent was removed under a reduced pressure to obtain a crude 6-fluoro-5-(4-hexylphenyl)-2-(4-octyloxyphenyl)pyridine-N-oxide.

After recrystallized from ethanol, it was subjected to silica gel column chromatography (eluent: ethyl acetate) and further recrystallized from heptane to obtain 15 mmol of the objective product.

Example 4

Preparation of 5-(4-propylcyclohexyl)-2-(3,4-difluorophenyl)pyridine-N-oxide

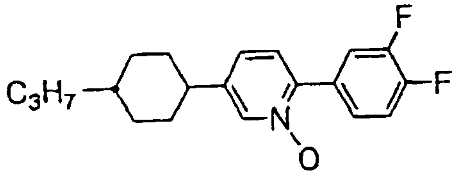

Mixture of 10 mmol of the 5-(4-propylcyclohexyl)-2-(3,4-difluorophenyl)pyridine prepared according to the method of Goto et al. (EP 240,320), 10 ml of hydrogen peroxide solution, and 60 ml of acetic acid was stirred at 80°C for 4 hours. Then, 7 ml of hydrogen peroxide solution was added thereto and further stirred at the same temperature for 8 hours. The reaction solution was concentrated under a reduced pressure, the residue was dissolved in chloroform, the organic layer was washed with 20 % aqueous sodium hydroxide solution, and the solvent was removed under a reduced pressure to obtain a crude 5-(4-propylcyclohexyl)-2-(3,4-difluorophenyl)pyridine-N-oxide.

After recrystallized from ethanol, it was subjected to silica gel column chromatography (eluent: ethyl acetate) and further recrystallized from heptane to obtain 9 mmol of the objective product.

Example 5 (Use Example 2)

Liquid crystal composition (C) comprising

| | |
|---|---|
| 4-(4-propylcyclohexyl)benzonitrile | 24 % |
| 4-(4-pentylcyclohexyl)benzonitrile | 36 % |
| 4-(4-heptylcyclohexyl)benzonitrile | 25 % |
| 4'-(4-pentylcyclohexyl)-4-cyanobiphenyl | 15 % |

exhibited the following physical properties:

NI: 71.7°C　　$\Delta\varepsilon$: 11.0

Liquid crystal composition (D) comprising 85 % of the liquid crystal composition (C) and 15 % of the 5-(4-propylcyclohexyl)-2-(3,4-difluorophenyl)pyridine-N-oxide obtained in Example 4 had the following physical property values. Values in the parentheses indicate the ones of 5-(4-propylcyclohexyl)-2-(3,4-difluorophenyl)pyridine-N-oxide calculated by extrapolation.

NI: 70.3°C (66.1°C)　　$\Delta\varepsilon$: 11.0 (11.0)

Following compounds were prepared by a method similar to that of Example 1. $\Delta\varepsilon$ indicates the extrapolated value of dielectric anisotropy determined at 25°C. In the following, the symbols and values in the parentheses show the type of mother liquid crystal and the mixing ratio with the compound of the general formula (1).

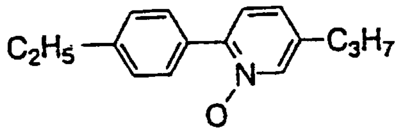

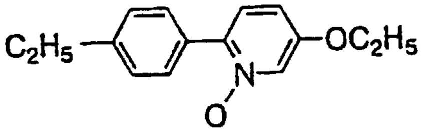

– 4.83 (A, 10%)

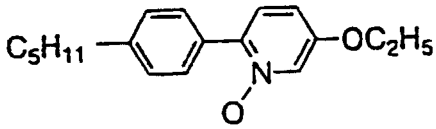

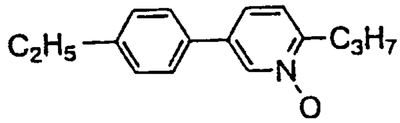

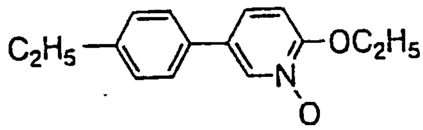

– 6.0 (A, 10%)

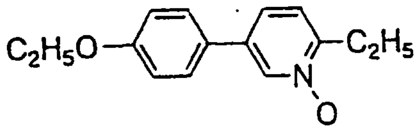

– 3.5 (A, 10%)

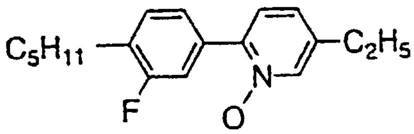

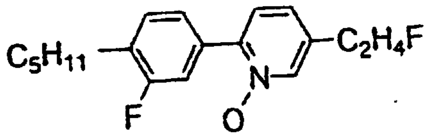

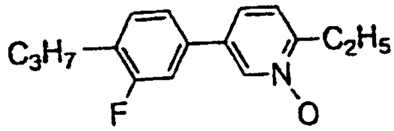

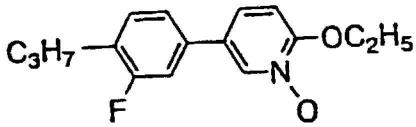

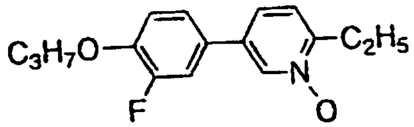

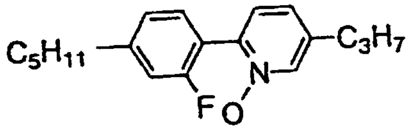

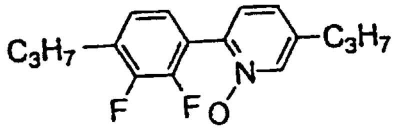

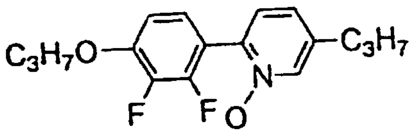

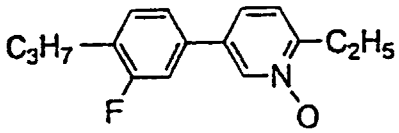

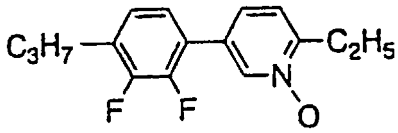

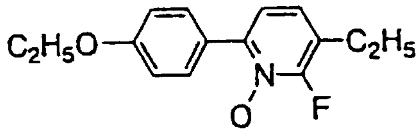

– 9.6 (A, 10%)

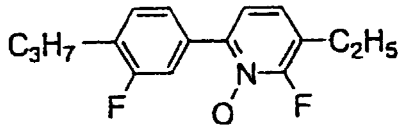

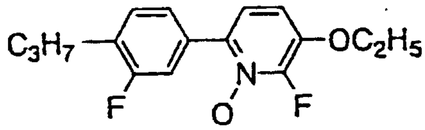

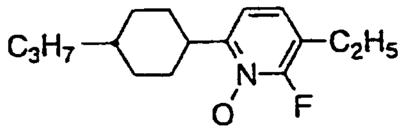

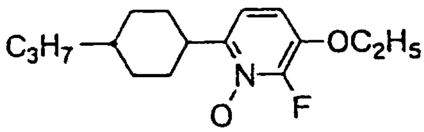

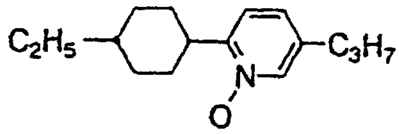

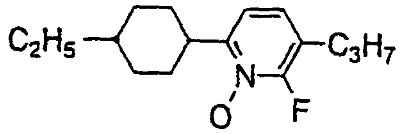

- 8.9 (A, 10%)

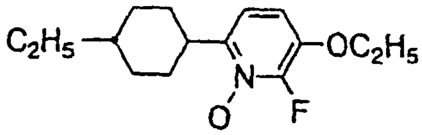

- 8.1 (A, 10%)

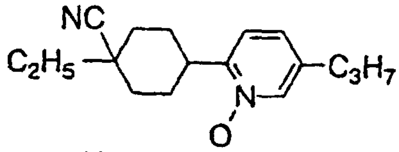

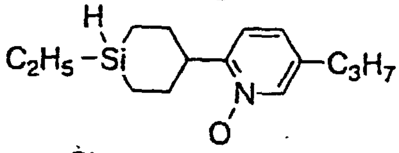

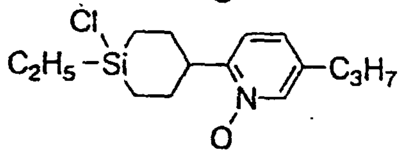

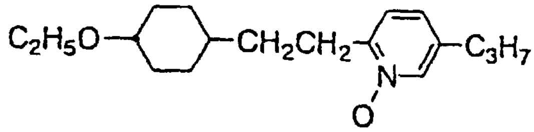

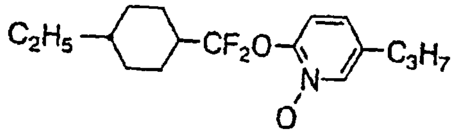

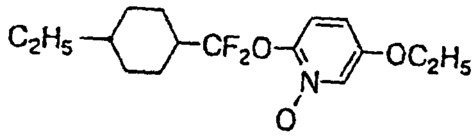

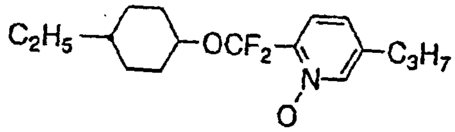

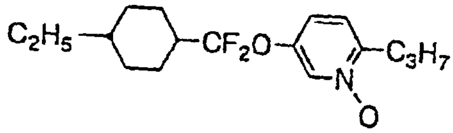

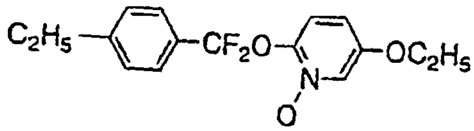

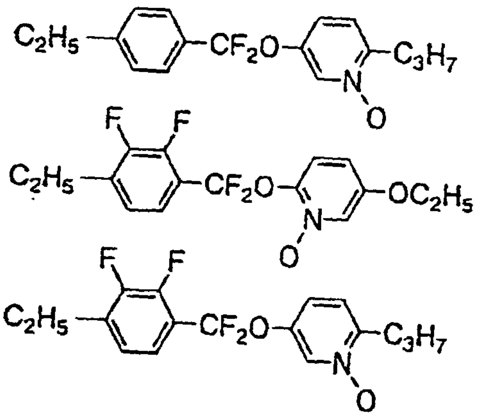

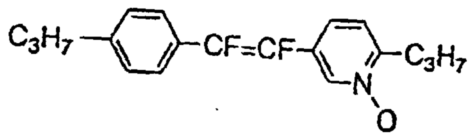

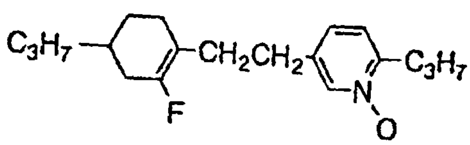

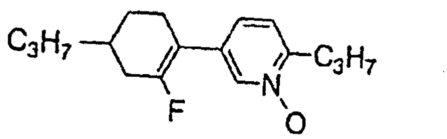

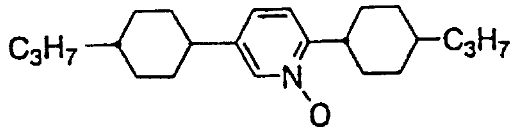

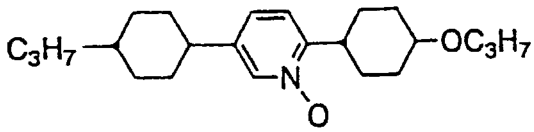

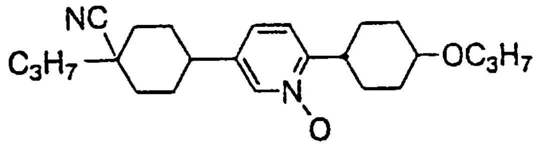

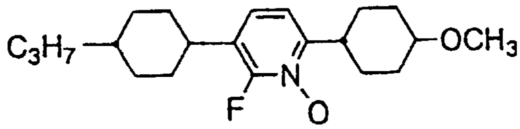

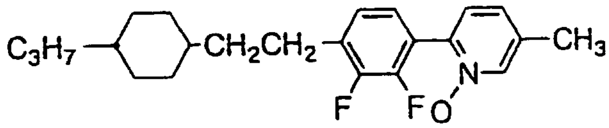

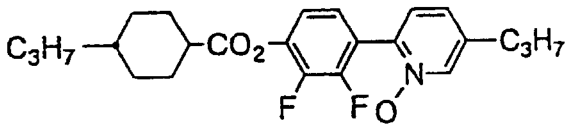

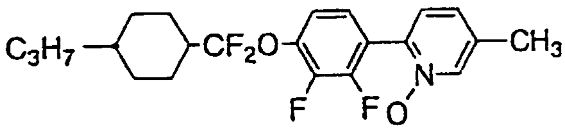

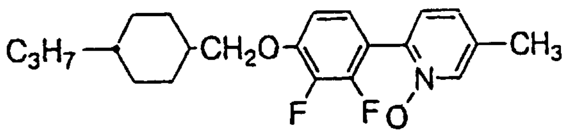

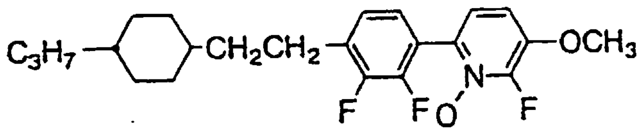

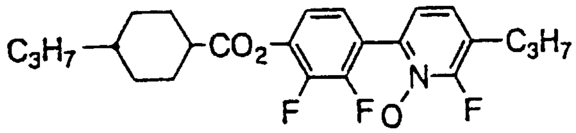

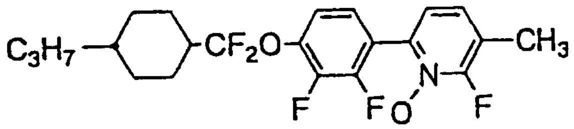

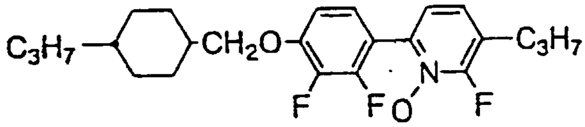

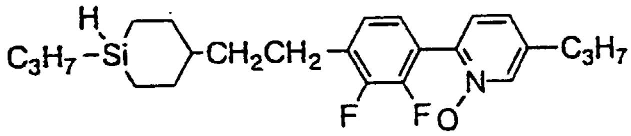

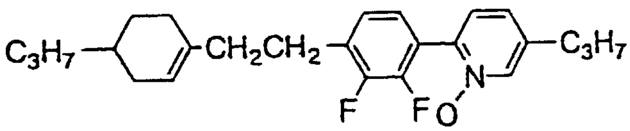

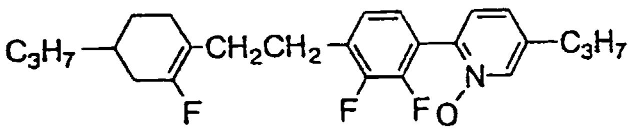

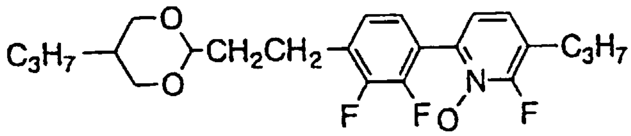

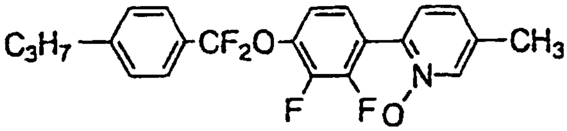

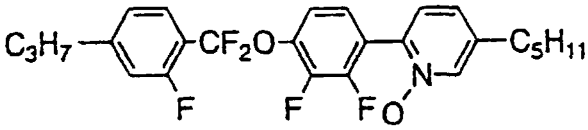

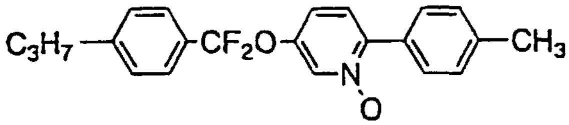

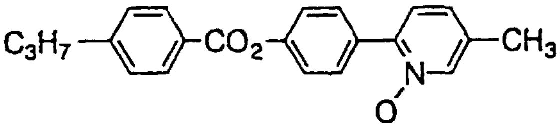

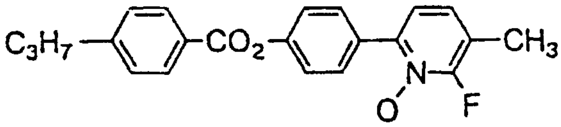

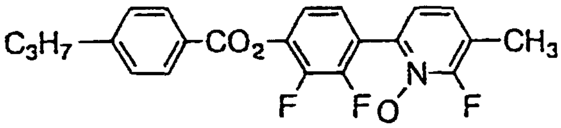

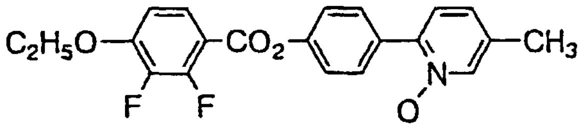

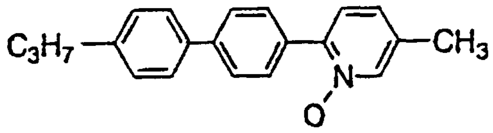

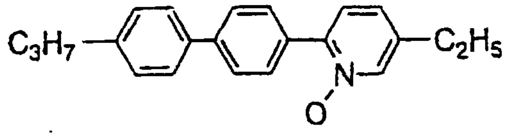

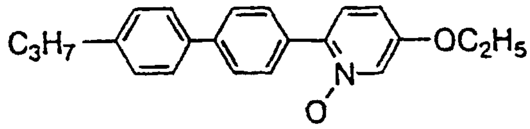

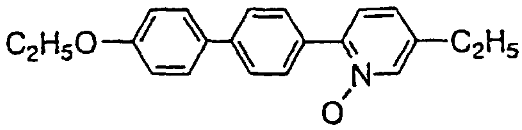

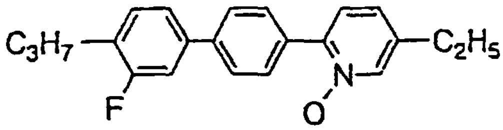

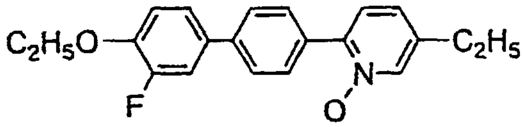

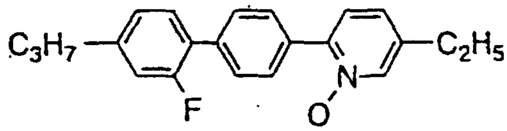

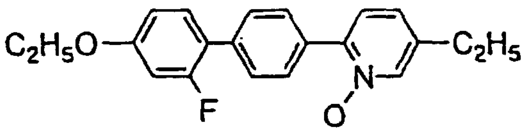

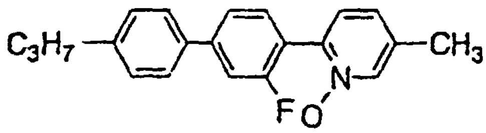

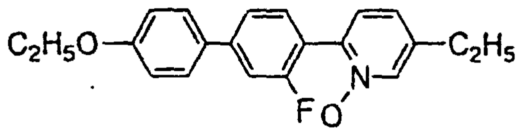

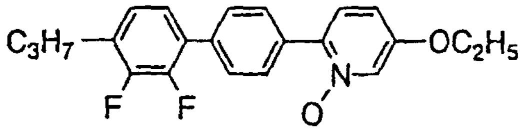

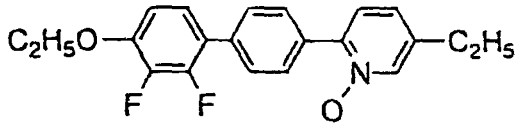

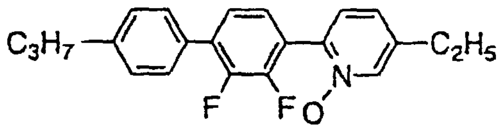

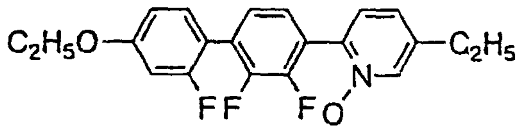

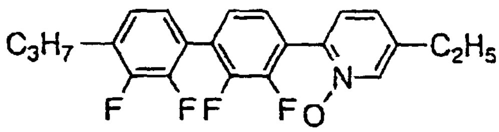

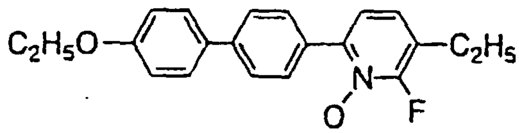

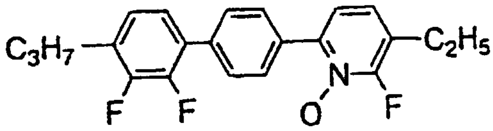

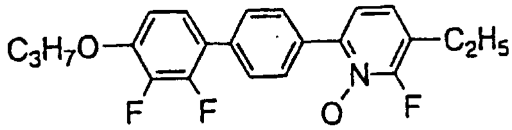

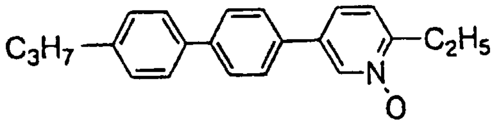

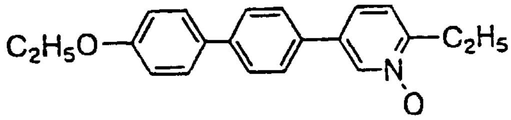

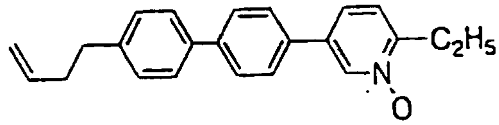

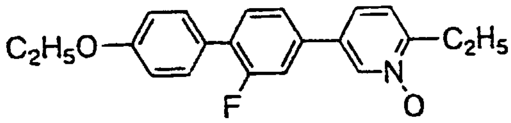

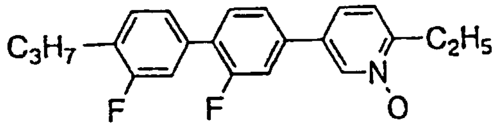

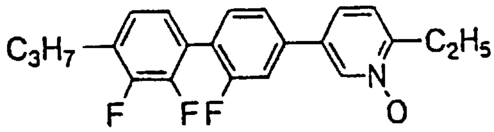

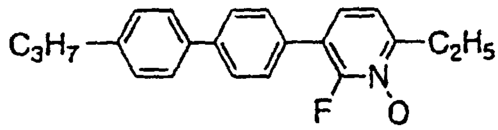

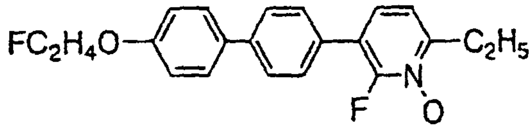

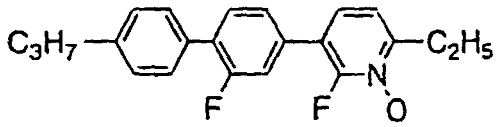

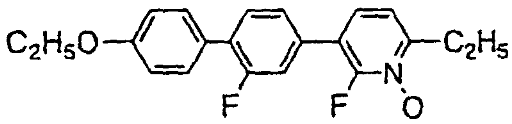

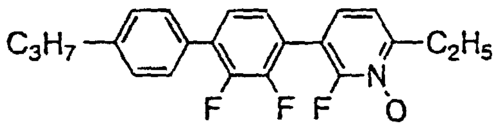

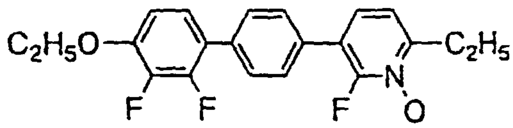

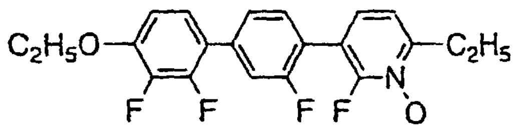

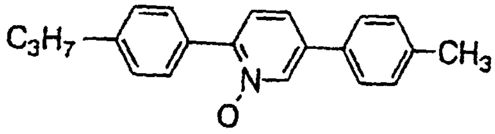

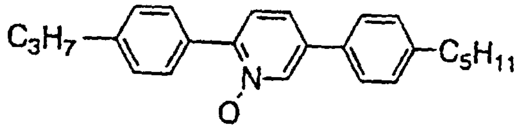

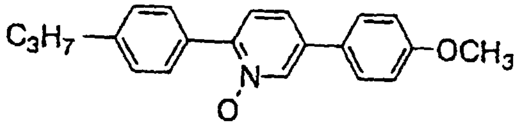

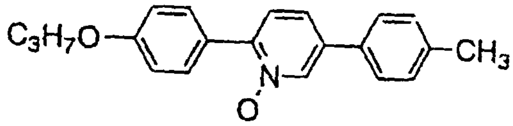

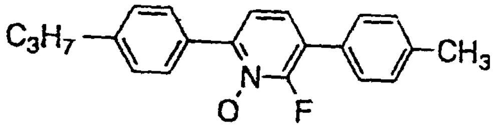

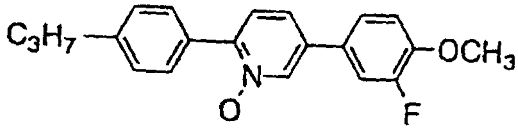

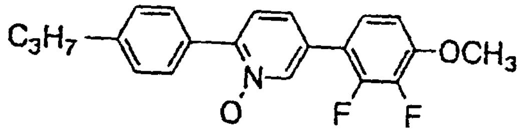

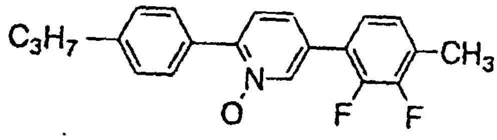

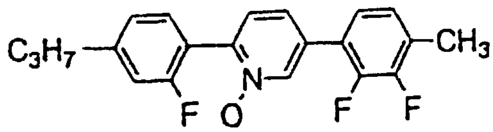

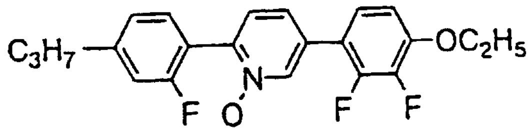

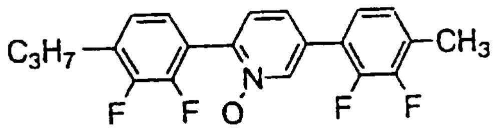

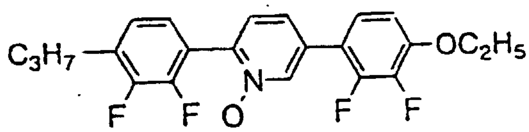

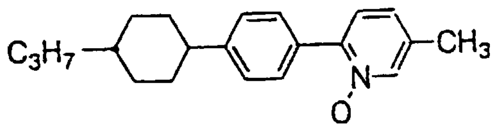

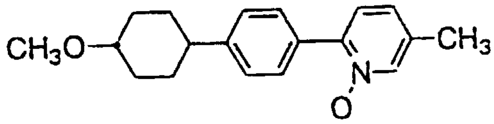

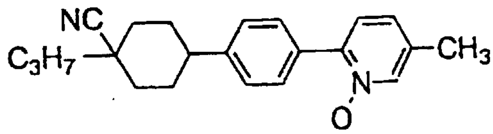

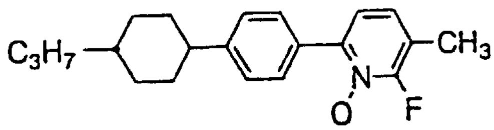

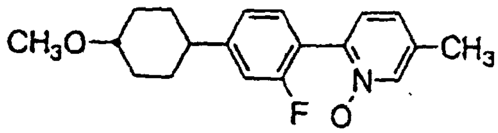

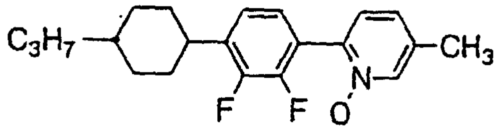

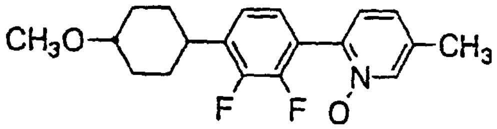

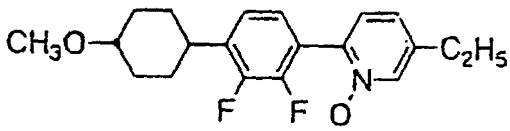

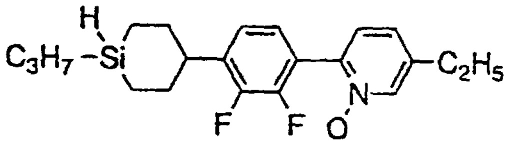

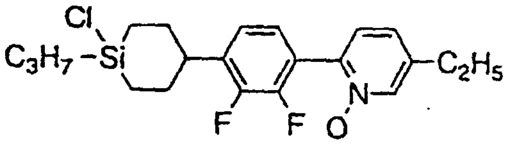

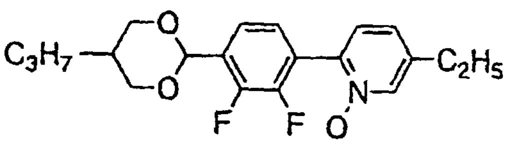

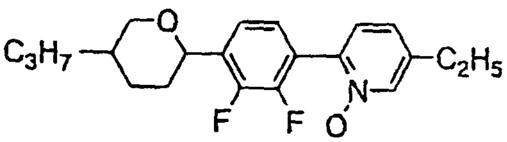

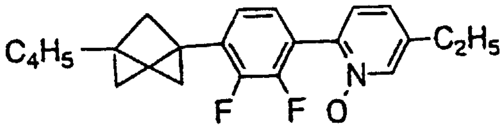

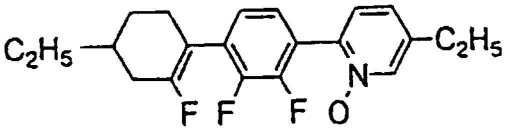

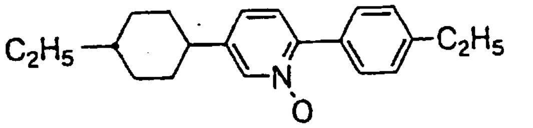

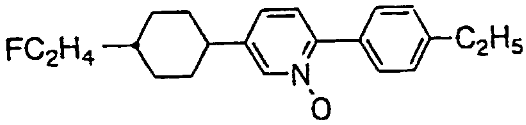

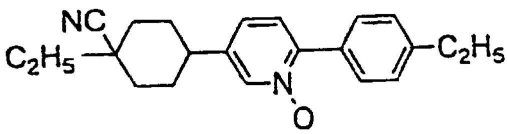

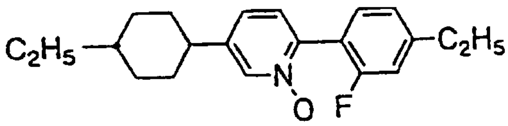

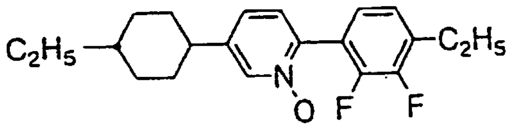

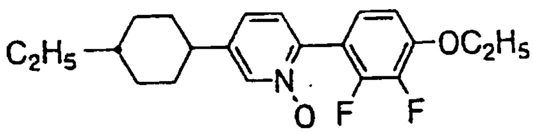

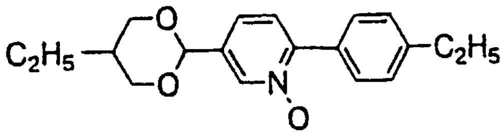

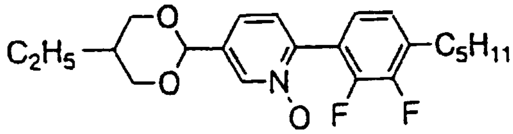

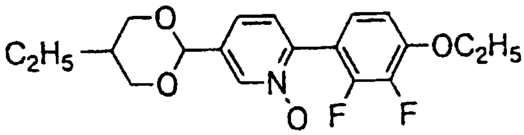

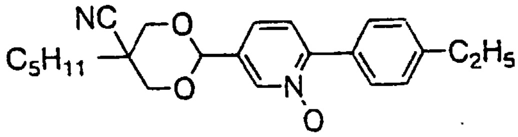

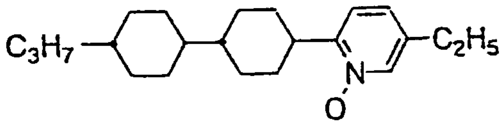

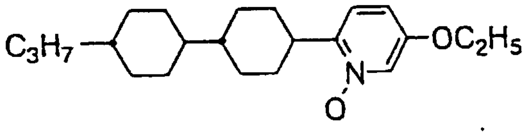

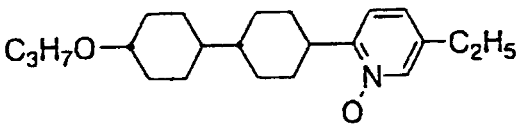

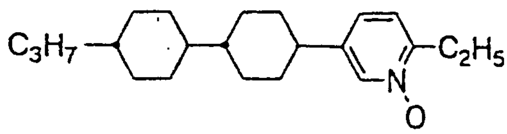

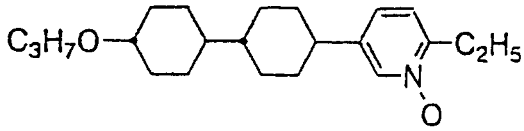

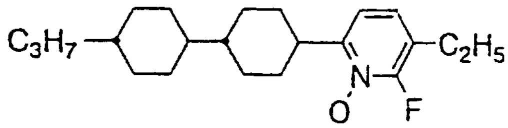

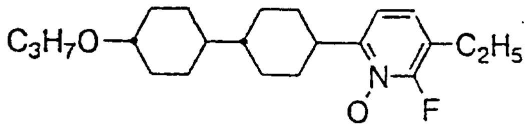

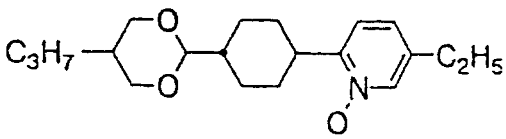

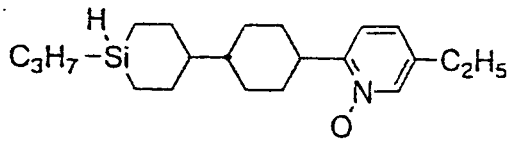

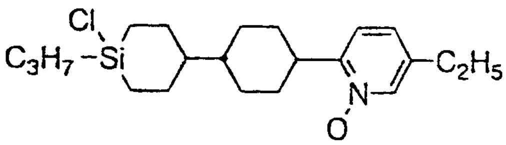

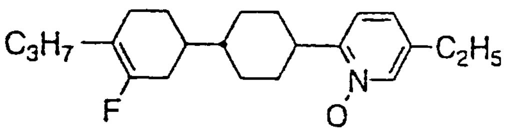

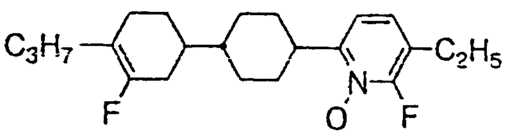

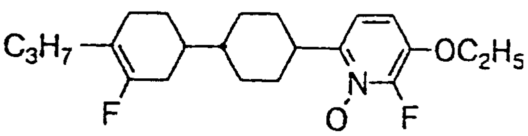

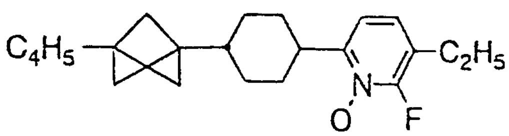

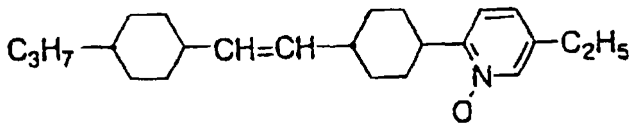

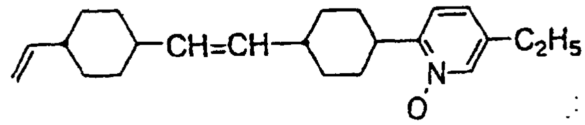

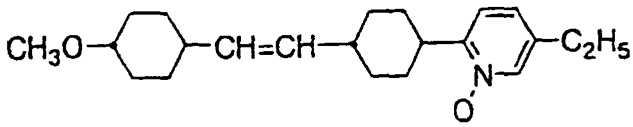

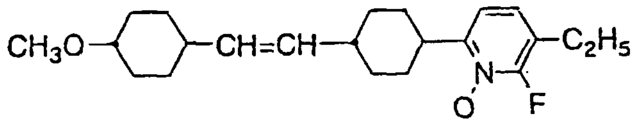

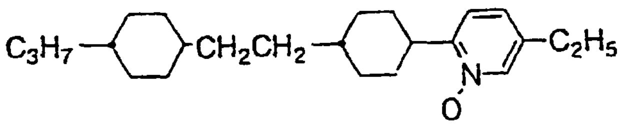

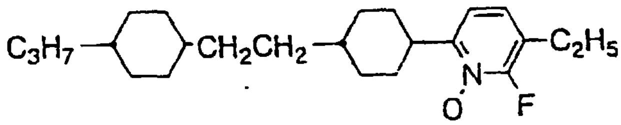

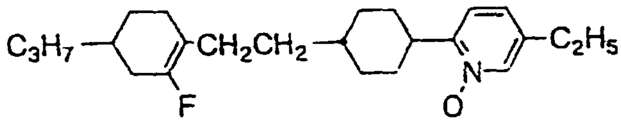

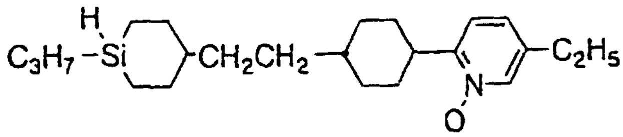

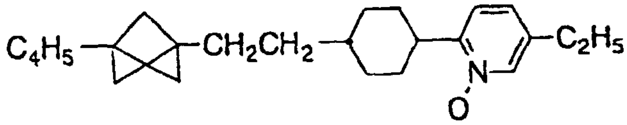

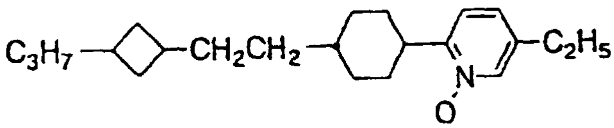

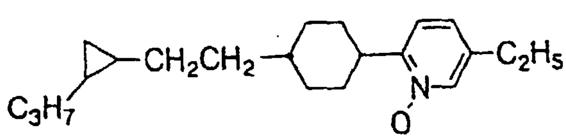

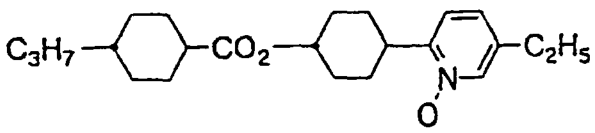

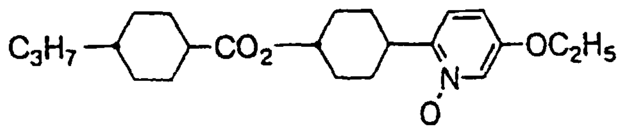

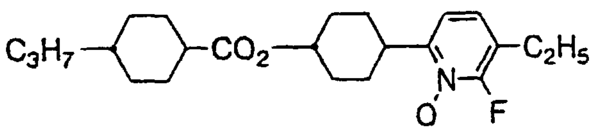

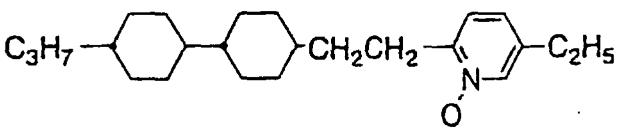

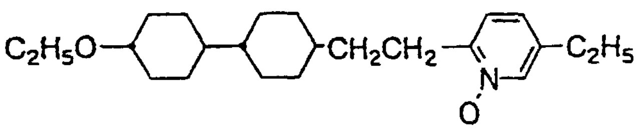

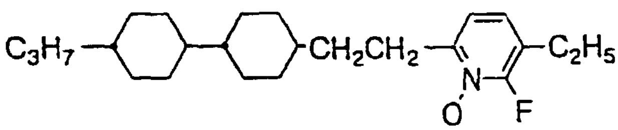

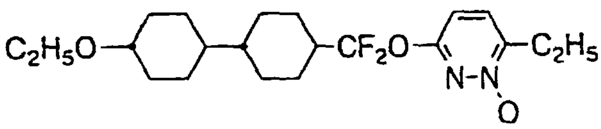

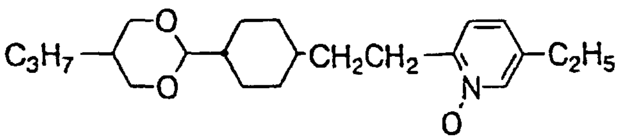

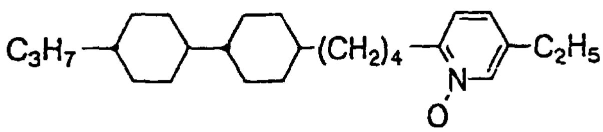

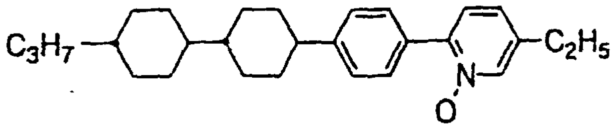

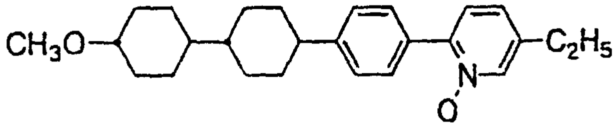

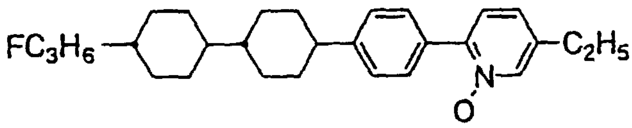

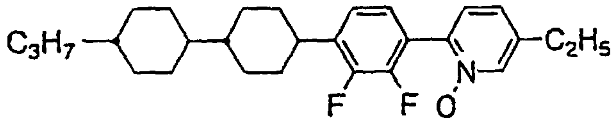

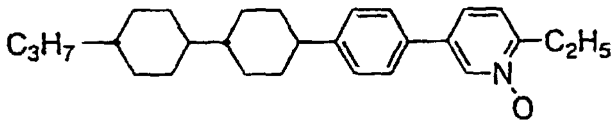

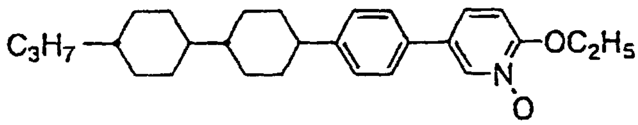

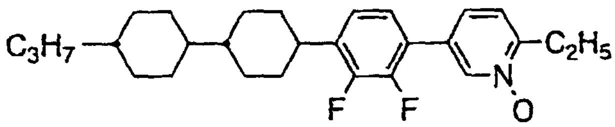

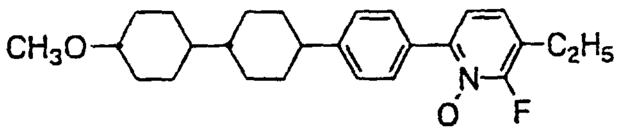

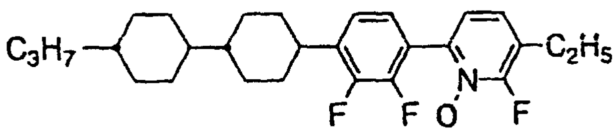

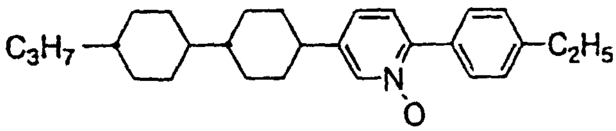

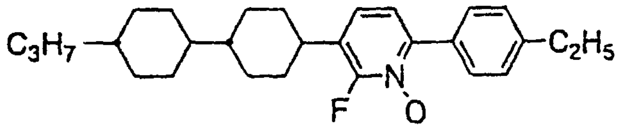

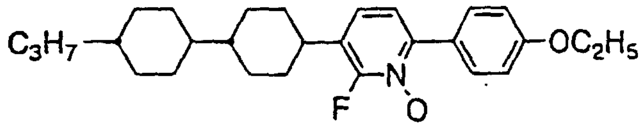

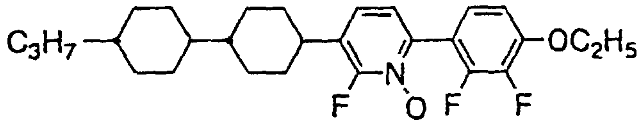

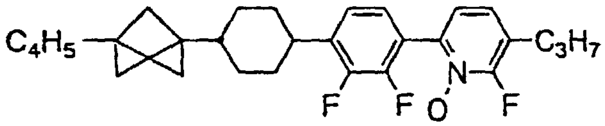

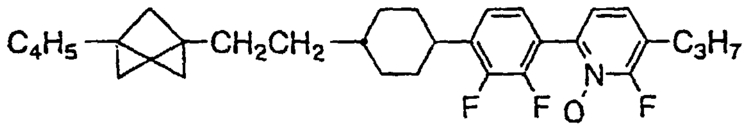

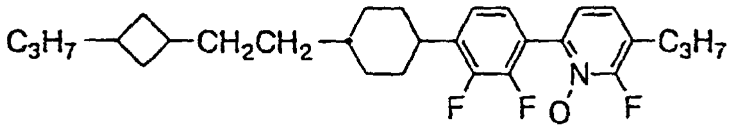

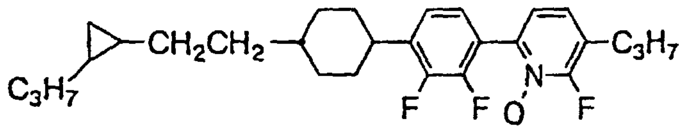

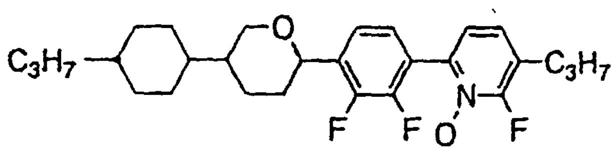

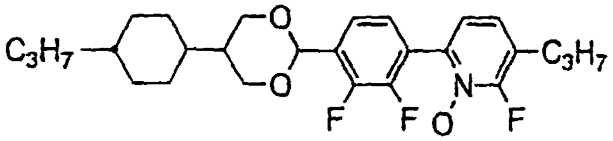

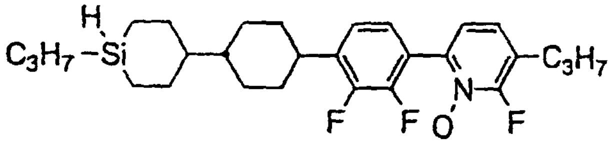

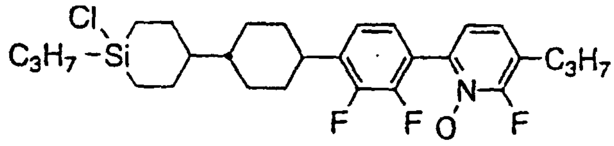

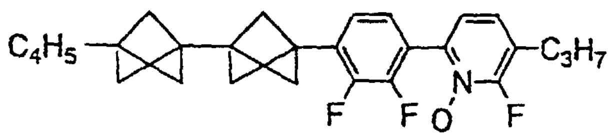

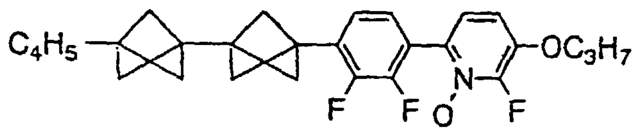

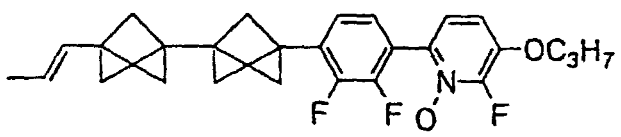

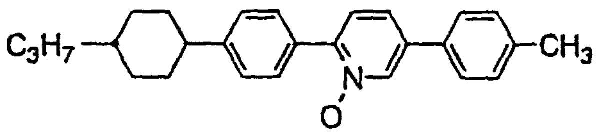

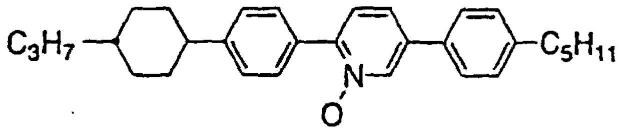

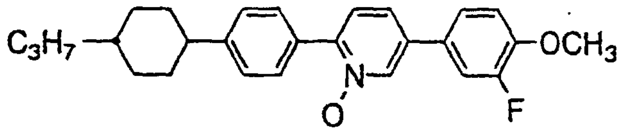

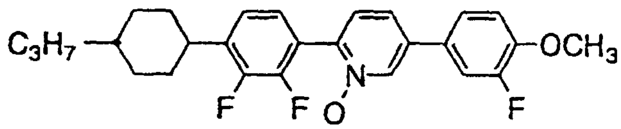

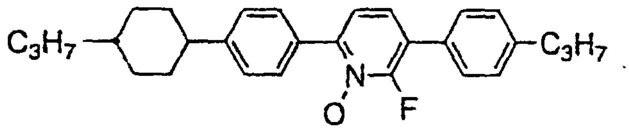

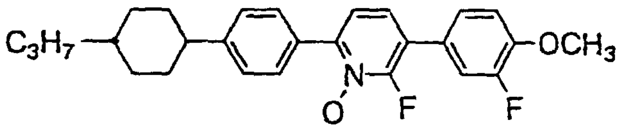

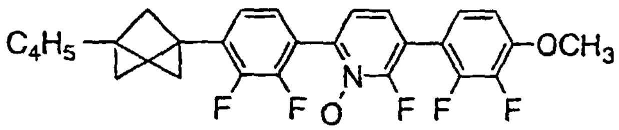

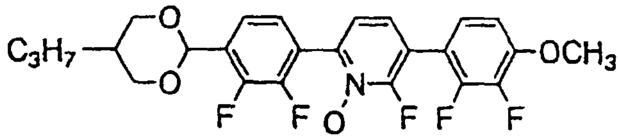

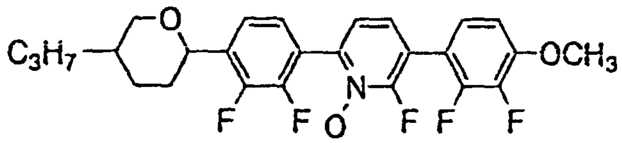

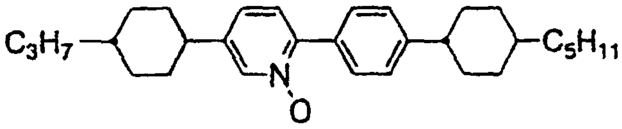

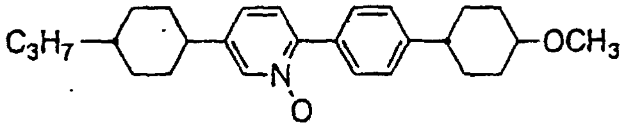

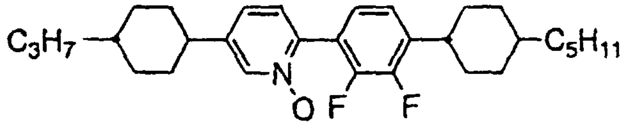

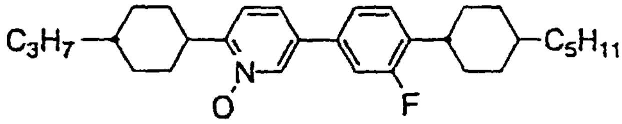

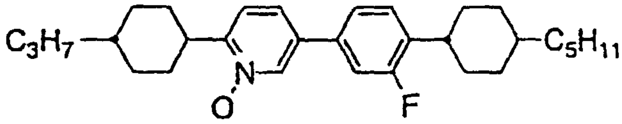

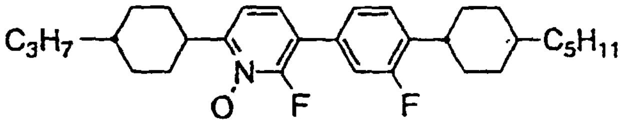

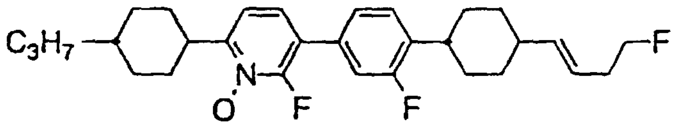

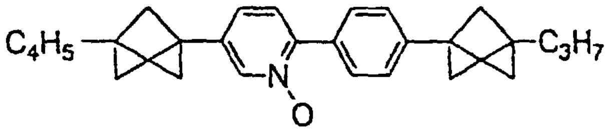

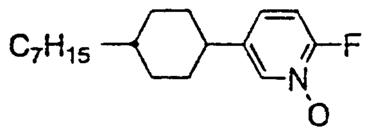

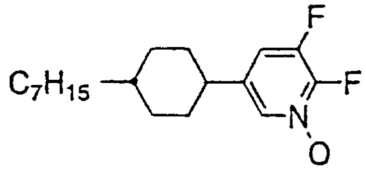

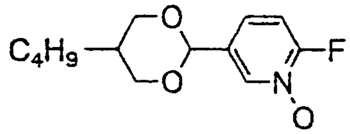

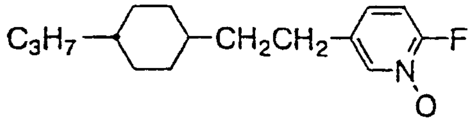

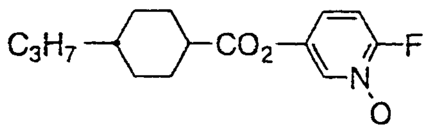

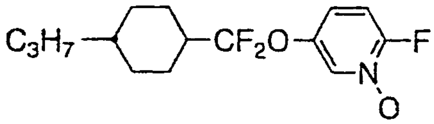

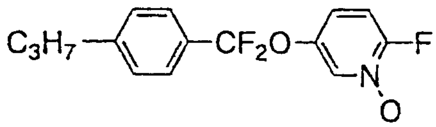

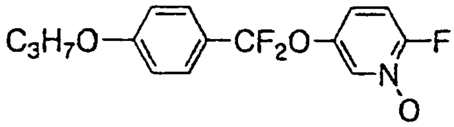

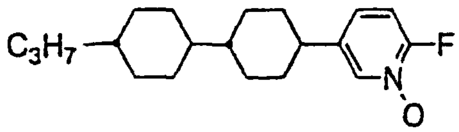

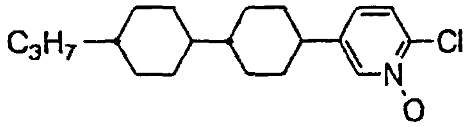

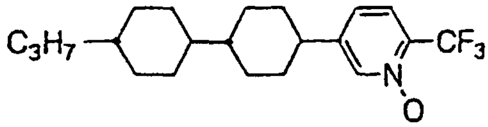

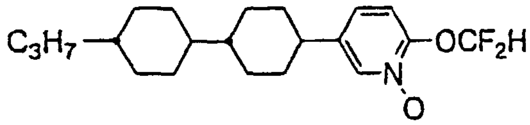

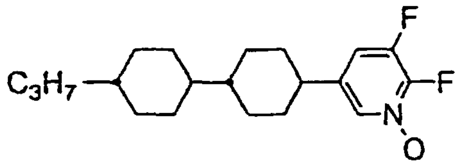

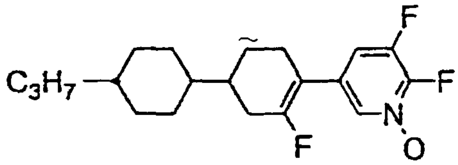

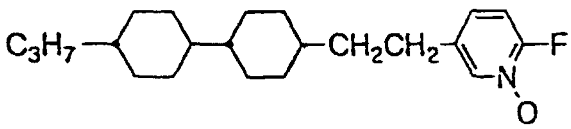

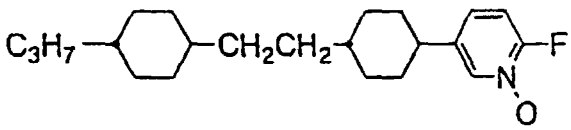

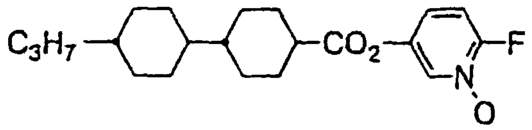

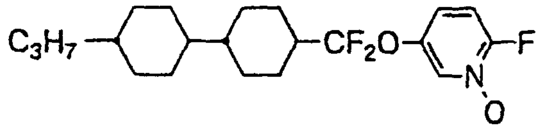

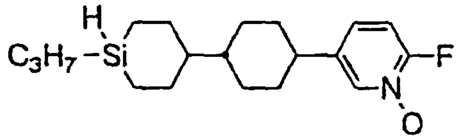

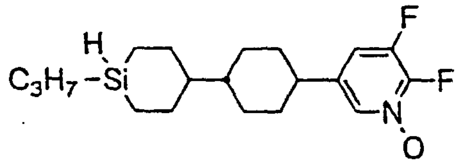

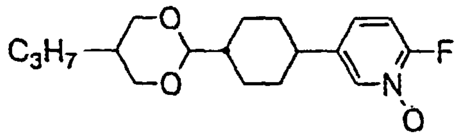

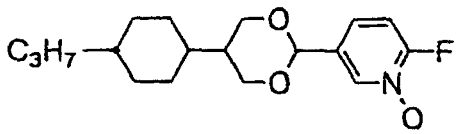

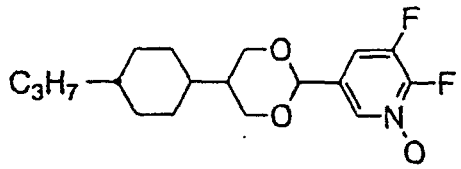

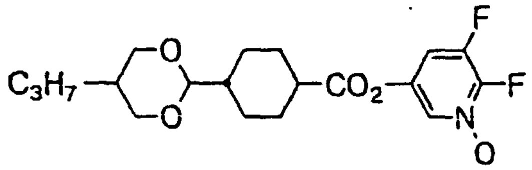

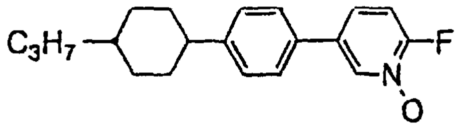

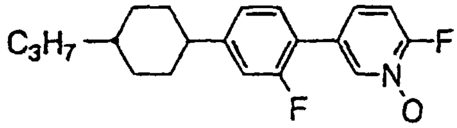

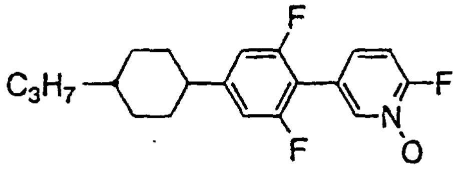

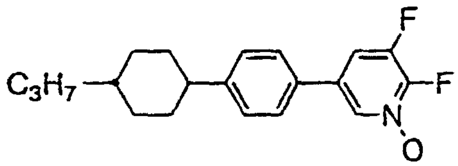

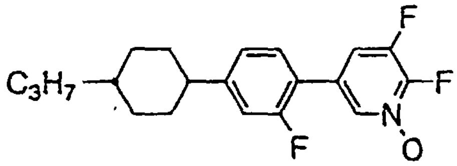

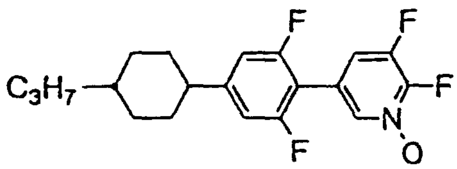

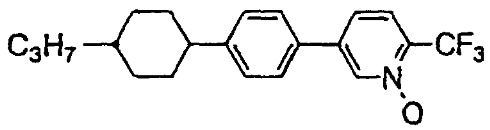

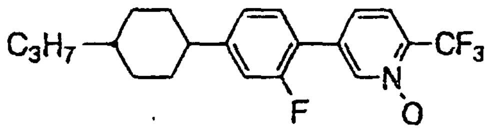

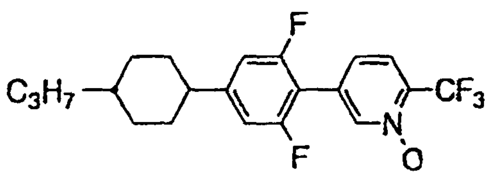

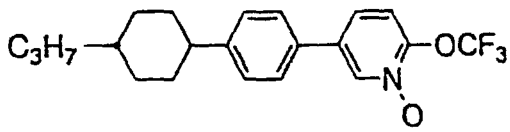

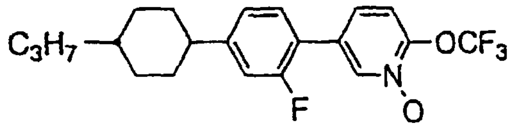

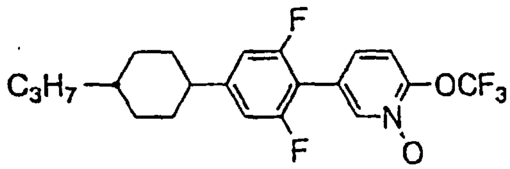

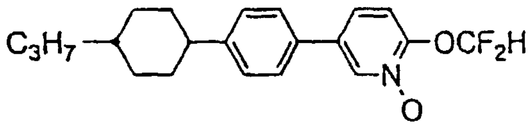

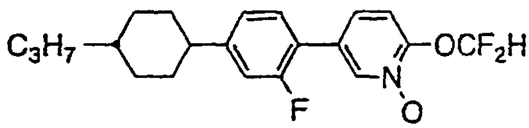

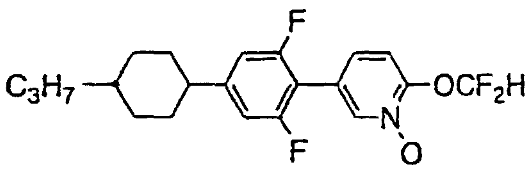

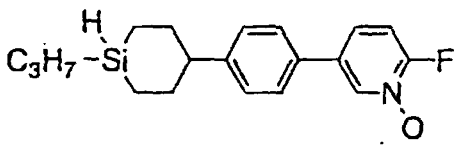

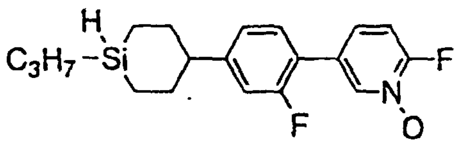

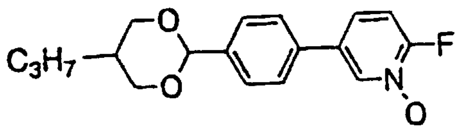

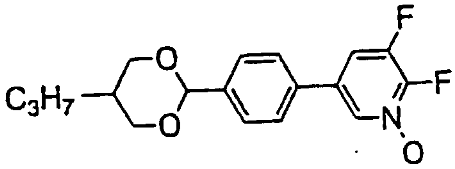

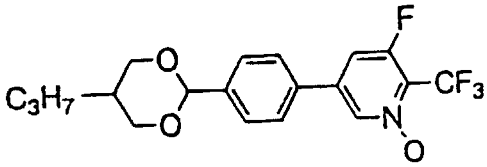

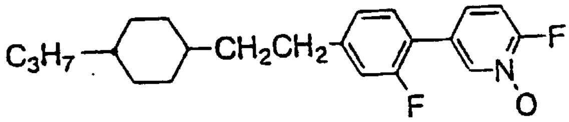

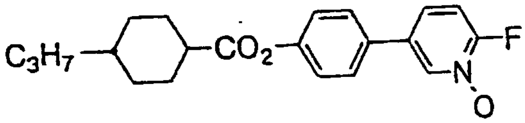

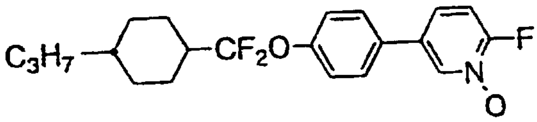

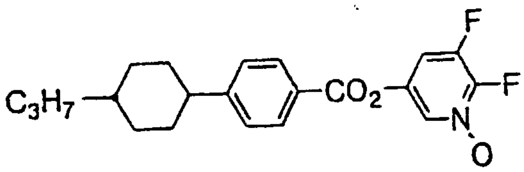

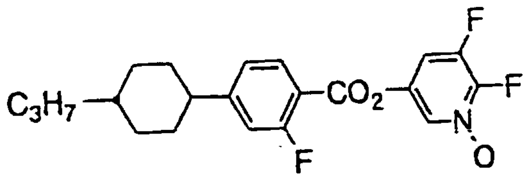

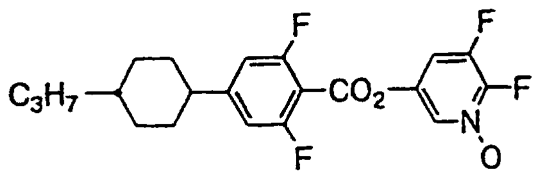

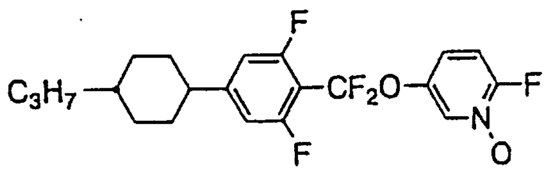

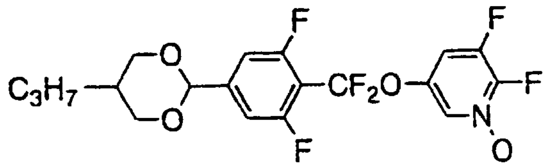

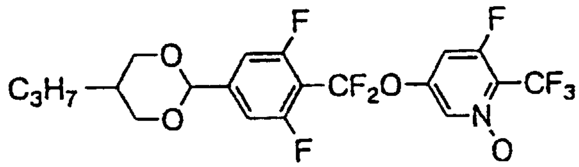

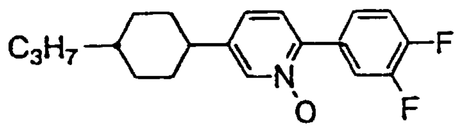

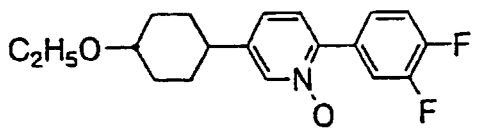

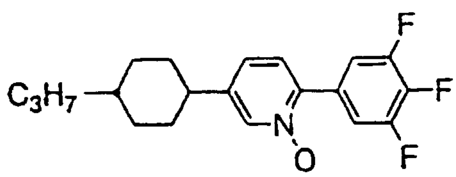

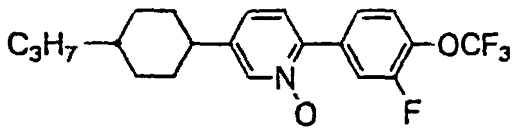

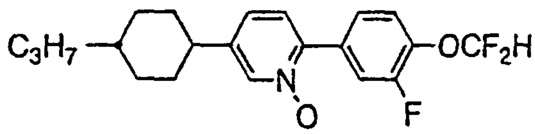

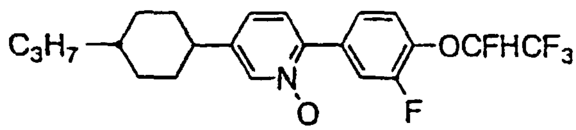

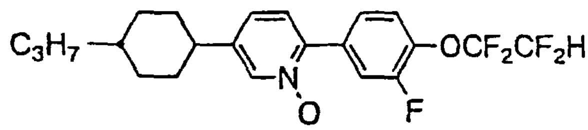

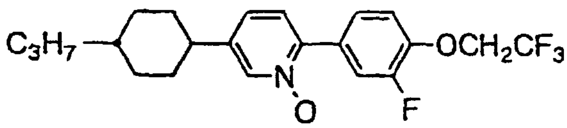

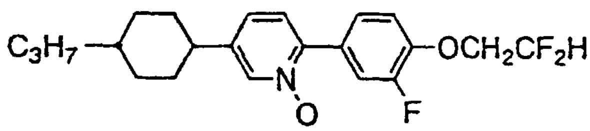

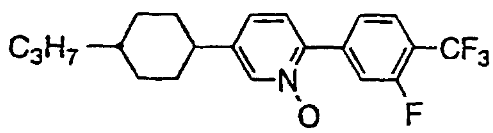

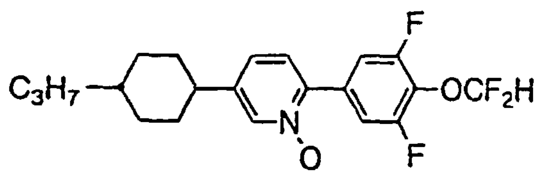

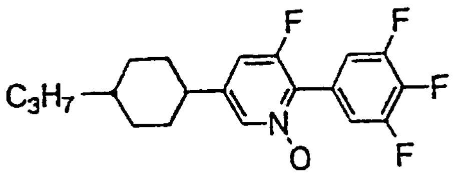

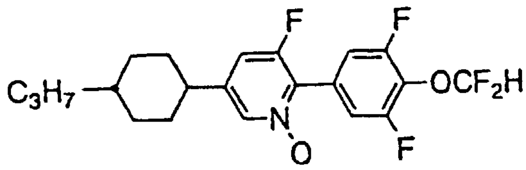

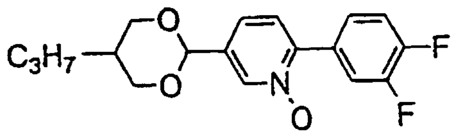

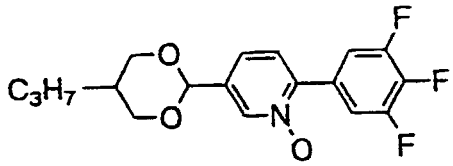

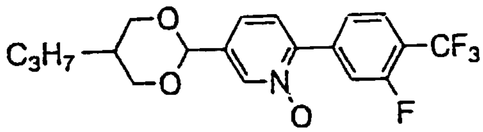

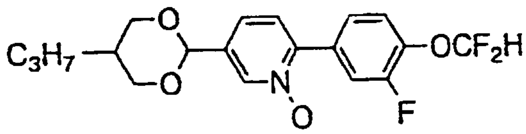

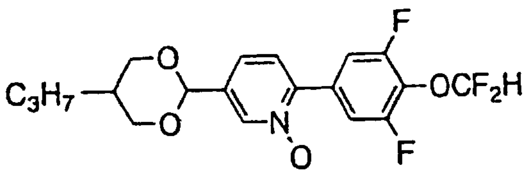

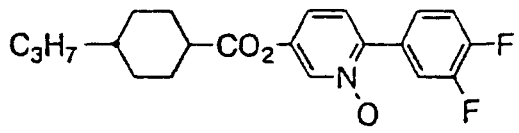

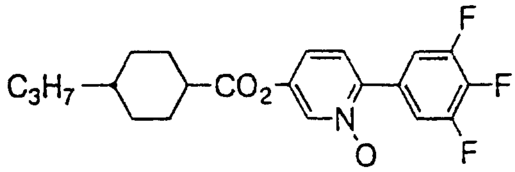

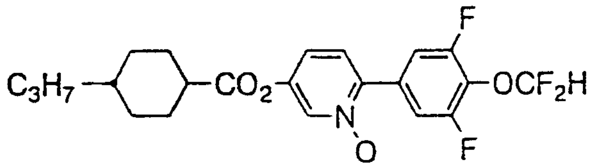

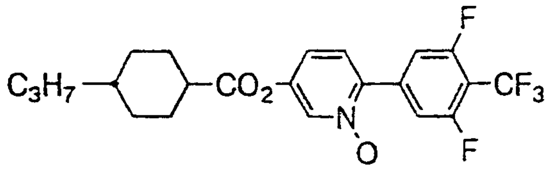

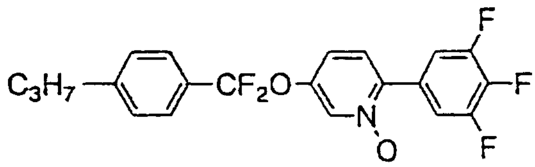

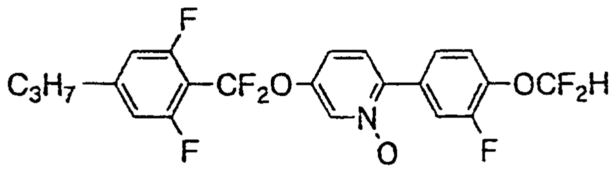

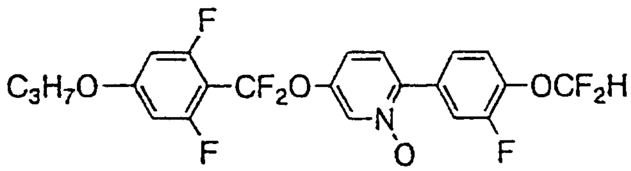

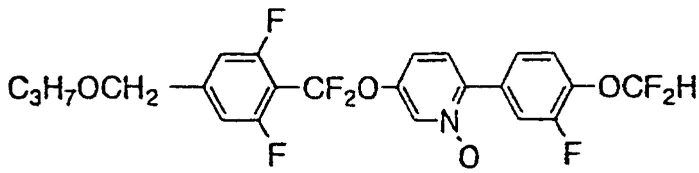

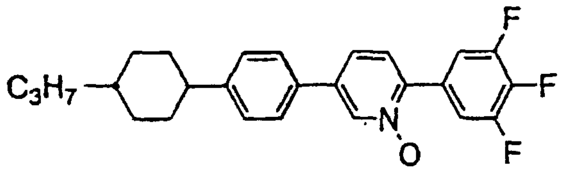

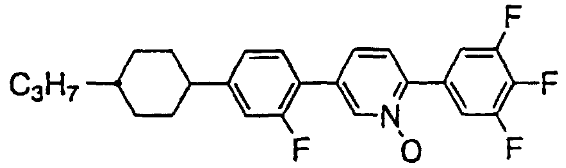

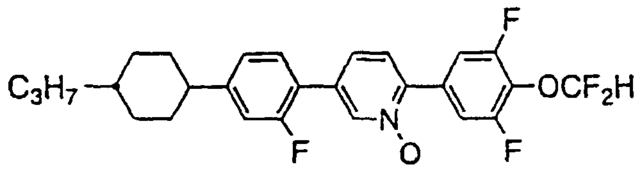

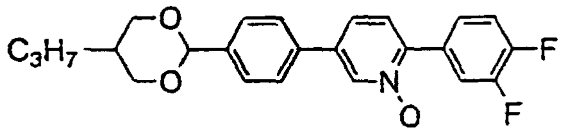

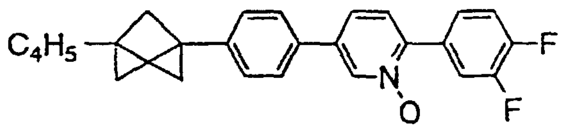

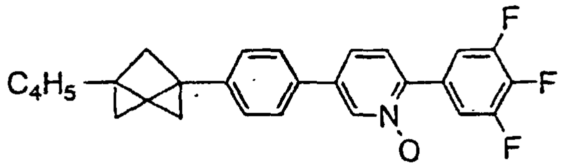

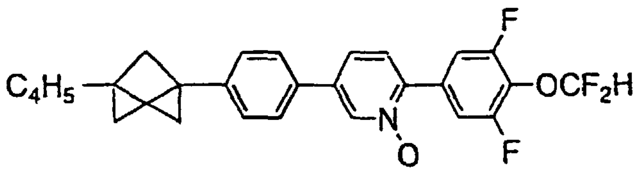

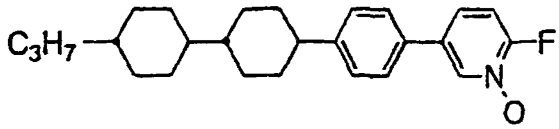

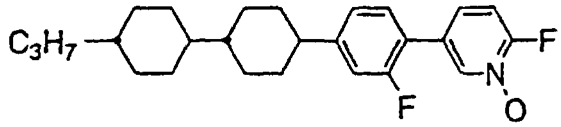

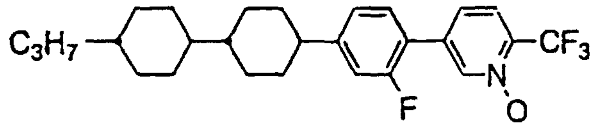

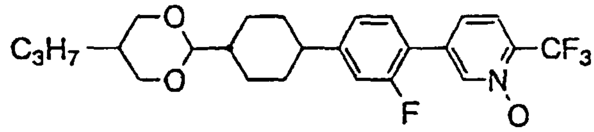

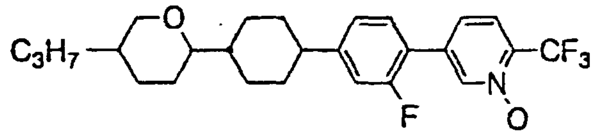

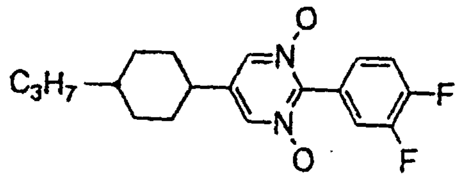

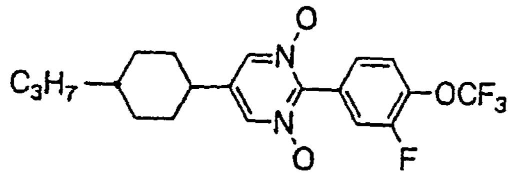

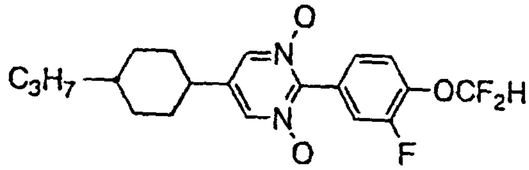

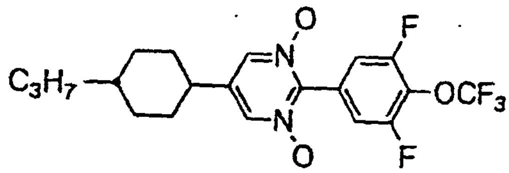

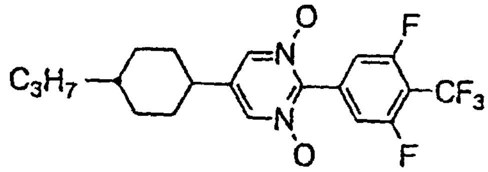

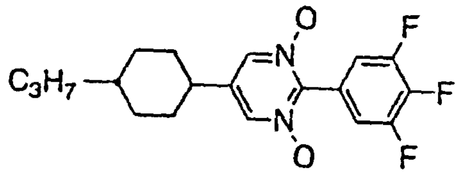

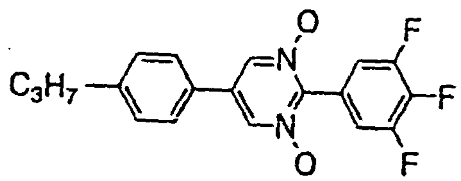

+ 37 ( C, 15% )

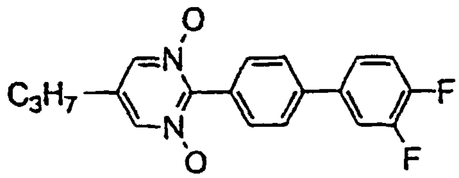

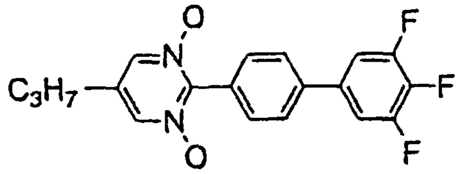

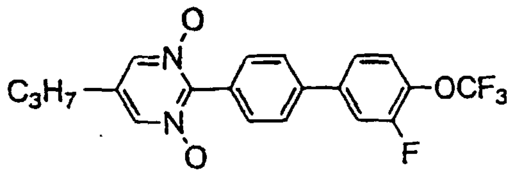

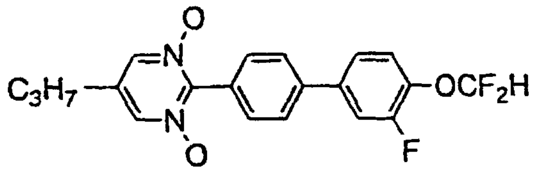

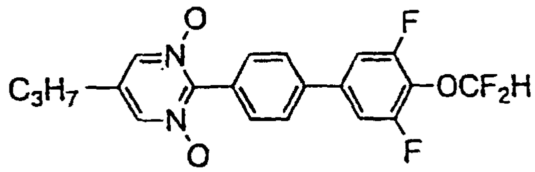

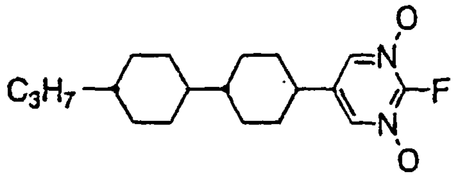

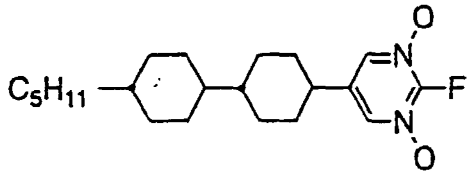

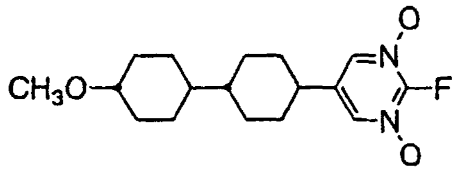

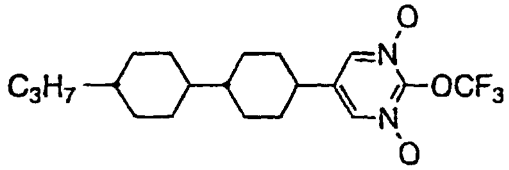

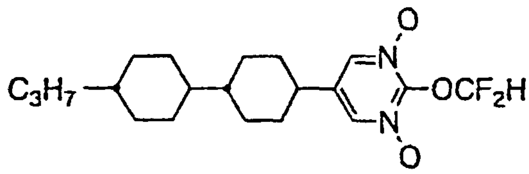

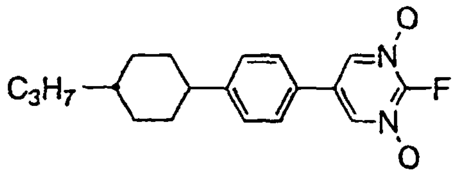

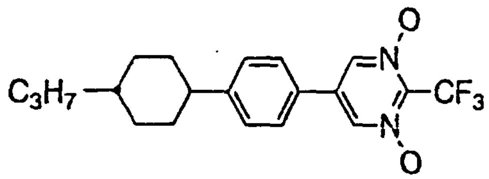

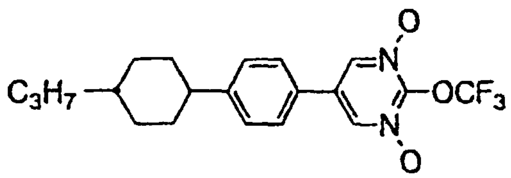

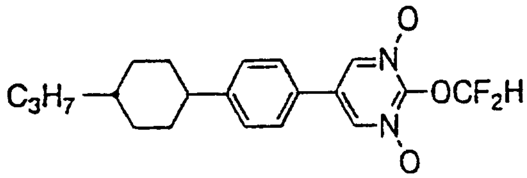

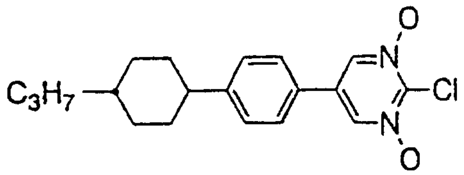

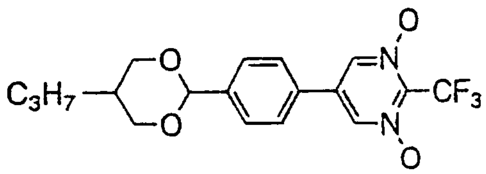

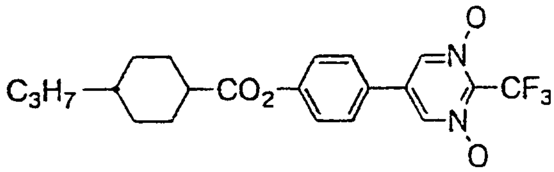

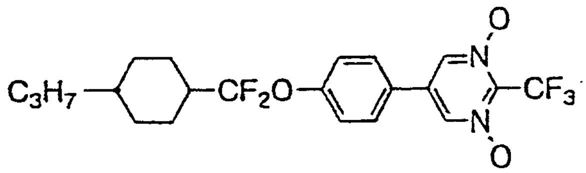

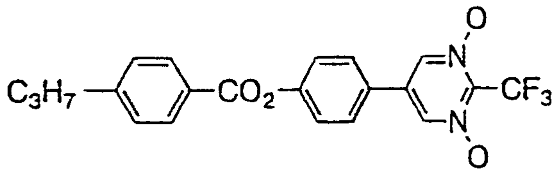

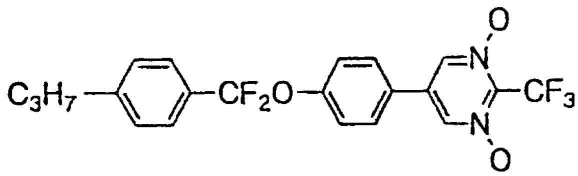

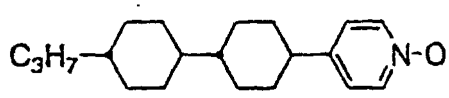

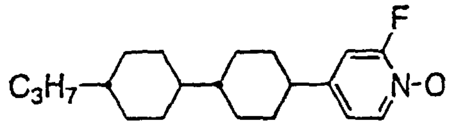

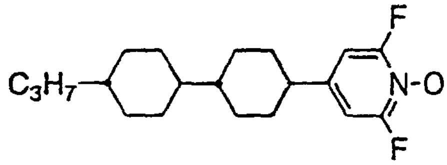

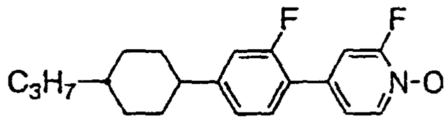

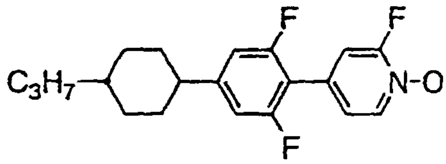

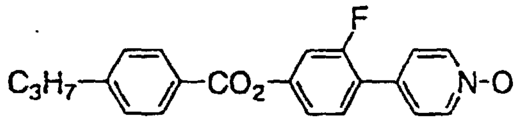

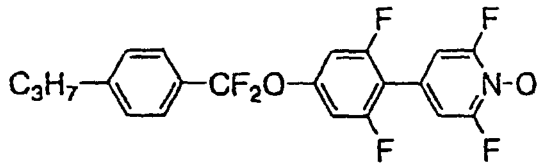

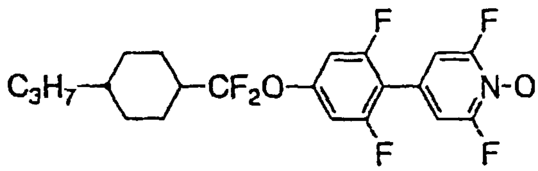

Example 6 (Comparative Example 1)

Pyridine derivative (Comparative compound 1), and a typical compound (Comparative compound 2) having a negative dielectric anisotropy were prepared according to the method of S. M. Kelly, Ferroelectrics, 180, 269 (1996) and V. Reiffenrath (DE-OS 3,807,917 and DE-OS 3,906,051), respectively, and their dielectric anisotropy value (extrapolated values) were calculated by using mother liquid crystal A. As a result, it was confirmed that the (absolute) value of dielectric anisotropy of the compound of Example 1 was remarkably large compared with the values of these Comparative compounds.

| | | Dielectric anisotropy value (Extrapolated value) |
|---|---|---|
| Compound of Example 1 | 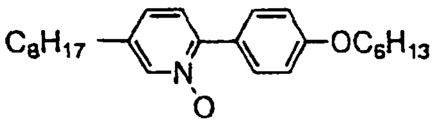 | -4.93 |
| Comparative compound 1 | 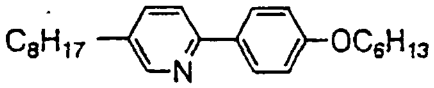 | 0.58 |
| Comparative compound 2 | 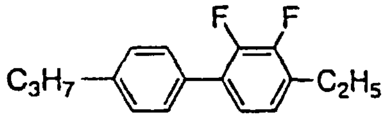 | -3.1 |

INDUSTRIAL APPLICABILITY

Liquid crystalline compounds of the present invention have an extremely large dielectric anisotropy value. Their dependency on temperature is vary small and their miscibility with other liquid crystal materials is also excellent. Further, novel liquid crystalline compounds having desired physical properties can be provided by selecting proper substituents in the compounds of the present invention.

Accordingly, liquid crystal compositions exhibiting a low threshold voltage and thus low driving voltage can be actualized by using the liquid crystalline compounds of the present invention as component of liquid crystal compositions. Further, excellent liquid crystal display devices fabricated by using the liquid crystal composition can be provided.

**Claims**

1. A liquid crystalline compound expressed by the general formula (1)

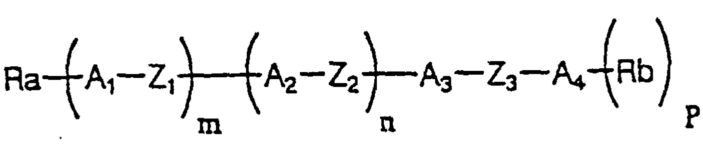 (1)

wherein Ra represents a straight chain or branched alkyl group having 1 to 20 carbon atoms in which groups any methylene group ($-CH_2-$) may be replaced by -O-, -CO-, -CH=CH-, -C≡C-, -SiRcRd-, cyclopropane-1,2-diyl, bicyclo[1.1.1]pentane-1,3-diyl, or cyclobutane-1,3-diyl, but there is not a case in which -O- continues, and one or more hydrogen atoms in Ra may be replaced by halogen atoms or cyano groups;

Rb represents a group selected from Ra, a halogen atom, or cyano group;
Rc and Rd each independently represent hydrogen atom or an alkyl group having 1 to 3 carbon atoms;
$A_1$, $A_2$, $A_3$, and $A_4$ each independently represent pyridine oxide, pyrimidine dioxide, trans-1,4-cyclohexylene, or 1,4-phenylene provided that at least one of $A_1$, $A_2$, $A_3$, and $A_4$ is pyridine oxide ring or pyrimidine dioxide ring, in these rings any $-CH_2-$ may be replaced by -O-, >CH- may be replaced by >SiRe-, and -CH= may be replaced by -N=, respectively, and one or two hydrogen atoms on the six-membered ring may be replaced by halogen atoms;
Re represents hydrogen atom, a halogen atom, or an alkyl group having 1 to 3 carbon atoms;
$Z_1$, $Z_2$, and $Z_3$ each independently represent single bond or an alkylene group having 1 to 4 carbon atoms one or more hydrogen atoms in which group may be replaced by halogen atoms, and any methylene group ($-CH_2-$) in which alkylene group may be replaced by -O-, -CO-, -CH=CH-, -C≡C-, or $-SiH_2-$, but there is not a case in

which -O- continues;

m, n, and p are each independently 0 or 1 provided that p is 0 when $A_4$ is pyridine oxide ring bonded, at its position 4, to $Z_3$; and

the atom which constitutes these compounds may be replaced by its isotope.

**2.** A liquid crystal composition comprising at least one liquid crystalline compound defined in claim 1.

**3.** A liquid crystal composition comprising, as a first component, at least one liquid crystalline compound defined in claim 1 and comprising, as a second component, at least one compound selected from the group consisting of the compounds expressed by the general formula (2), (3), or (4)

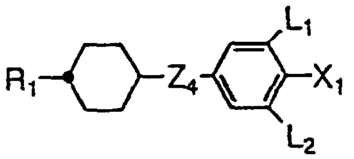

(2)

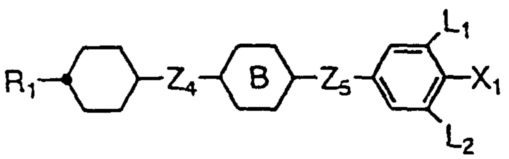

(3)

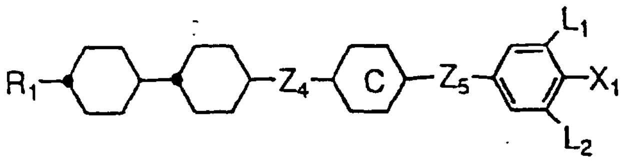

(4)

wherein $R_1$ represents an alkyl group having 1 to 10 carbon atoms in which group not-adjacent any methylene group may be replaced by -O- or -CH=CH-, and any hydrogen atom in which alkyl group may be replaced by fluorine atom;

$X_1$ represents fluorine atom, chlorine atom, $-OCF_3$, $-OCF_2H$, $-CF_3$, $-CF_2H$, $-CFH_2$, $-OCF_2CF_2H$, or $-OCF_2CFHCF_3$;

$L_1$ and $L_2$ each independently represent hydrogen atom or fluorine atom;

$Z_4$ and $Z_5$ each independently represent 1,2-ethylene, 1,4-butylene, -COO-, $-CF_2O-$, $-OCF_2-$, -CH=CH-, or single bond;

ring B represents trans-1,4-cyclohexylene, 1,3-dioxane-2,5-diyl, 1,4-phenyleyl hydrogen atom of which may be replaced by fluorine atom, or 1,4-phenylene hydrogen atom of which may be replaced by fluorine atom;

ring C represents trans-1,4-cyclohexylene, or 1,4-phenylene hydrogen atom of which may be replaced by fluorine atom; and

the atom which constitutes these compounds may be replaced by its isotope.

**4.** A liquid crystal composition comprising, as a first component, at least one liquid crystalline compound defined in claim 1, and comprising, as a second component, at least one compound selected from the group consisting of the compounds expressed by the general formula (5) or (6)

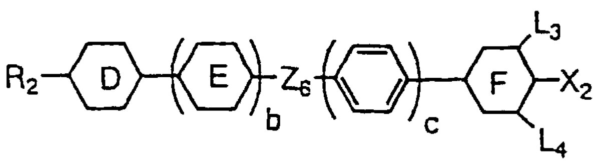

(5)

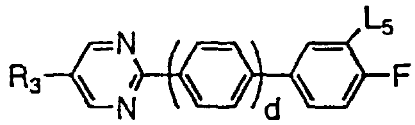
(6)

wherein $R_2$ and $R_3$ each independently represent an alkyl group having 1 to 10 carbon atoms in which group not-adjacent any methylene group may be replaced by -O- or -CH=CH-, and any hydrogen atom in which group may be replaced by fluorine atom;

$X_2$ represents -CN or -C≡C-CN;
ring D represents trans-1,4-cyclohexylene, 1,4-phenylene, 1,3-dioxane-2,5-diyl, or pyrimidine-2,5-diyl;
ring E represents trans-1,4-cyclohexylene or pyrimidine-2,5-diyl, or 1,4-phenylene hydrogen atom of which may be replaced by fluorine atom;
ring F represents trans-1,4-cyclohexylene or 1,4-phenylene;
$Z_6$ represents 1,2-ethylene, -COO-, or single bond;
$L_3$, $L_4$, and $L_5$ each independently represent hydrogen atom or fluorine atom;
b, c, and d are each independently 0 or 1; and
the atom which constitutes these compounds may be replaced by its isotope.

**5.** A liquid crystal composition comprising, as a first component, at least one liquid crystalline compound defined in claim 1, and comprising, as a second component, at least one compound selected from the group consisting of the compounds expressed by the general formula (7), (8), or (9)

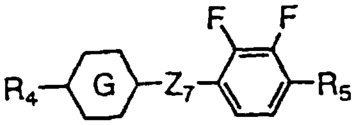
(7)

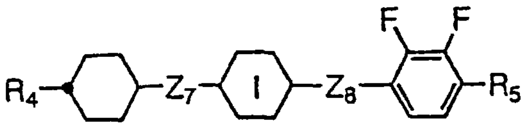
(8)

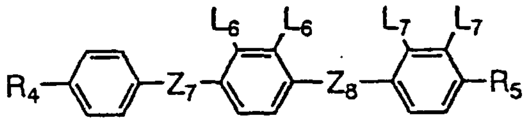
(9)

wherein $R_4$ and $R_5$ each independently represent an alkyl group having 1 to 10 carbon atoms in which alkyl group not-adjacent any methylene group my be replaced by -O- or -CH=CH-, and any hydrogen atom in which alkyl group my be replaced by fluorine atom;

ring G and ring I each independently represent trans-1,4-cyclohexylene or 1,4-phenylene;
$L_6$ and $L_7$ each independently represent hydrogen atom or fluorine atom, but there is not a case in which $L_6$ and $L_7$ simultaneously represent hydrogen atom;
$Z_7$ and $Z_8$ each independently represent 1,2-ethylene, -COO-, or single bond; and
the atom which constitutes these compounds may be replaced by its isotope.

**6.** A liquid crystal composition, comprising, as a first component, at least one liquid crystalline compound defined in

claim 1, comprising, as a second component, at least one compound selected from the group consisting of the compounds expressed by the general formula (2), (3), or (4) described above, and comprising, as a third component, at least one compound selected from the group consisting of the compounds expressed by the general formula (10), (11), or (12)

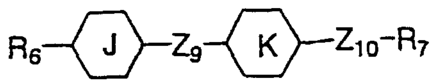

(10)

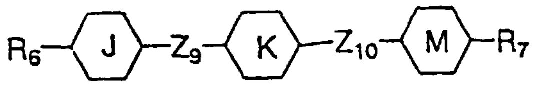

(11)

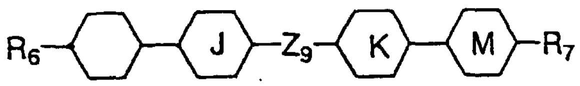

(12)

wherein $R_6$ and $R_7$ each independently represent an alkyl group having 1 to 10 carbon atoms in which alkyl group not-adjacent any methylene group may be replaced by -O- or -CH=CH-, and any hydrogen atom in which alkyl group may be replaced by fluorine atom;

ring J, ring K, and ring M each independently represent trans-1,4-cyclohexylene or pyrimidine-2,5-diyl, or 1,4-phenylene hydrogen atom of which may be replaced by fluorine atom;
$Z_9$ and $Z_{10}$ each independently represent 1,2-ethylene, -C≡C-, -COO-, -CH=CH-, or single bond; and
the atom which constitutes these compounds may be replaced by its isotope.

**7.** A liquid crystal composition comprising, as a first component, at least one liquid crystalline compound defined in claim 1, comprising, as a second component, at least one compound selected from the group consisting of the compounds expressed by the general formula (5) or (6) described above, and comprising, as a third component, at least one compound selected from the group consisting of the compounds expressed by the general formula (10), (11), or (12) described above.

**8.** A liquid crystal composition comprising, as a first component, at least one liquid crystalline compound defined in claim 1, comprising, as a second component, at least one compound selected from the group consisting of the compounds expressed by the general formula (7), (8), or (9) described above, and comprising, as a third component, at least one compound selected from the group consisting of the compounds expressed by the general formula (10), (11), or (12) described above.

**9.** A liquid crystal composition comprising, as a first component, at least one liquid crystalline compound defined in claim 1, comprising, as a second component, at least one compound selected from the group consisting of the compounds expressed by the general formula (2), (3), or (4) described above, comprising, as a third component, at least one compound selected from the group consisting of the compounds expressed by the general formula (5) or (6) described above, and comprising, as a fourth component, at least one compound selected from the group consisting of the compounds expressed by the general formula (10), (11), or (12) described above.

**10.** A liquid crystal composition further comprising one or more optical active compounds in addition to the liquid crystal composition defined in any one of claims 2 to 9.

**11.** A liquid crystal display device fabricated by using the liquid crystal composition defined in any one of claims 2 to 10.

# INTERNATIONAL SEARCH REPORT

International application No.
PCT/JP98/03012

A. CLASSIFICATION OF SUBJECT MATTER
Int.Cl$^6$ C07D213/89, C07D239/26, C09K19/24, C09K19/42, G02F1/13

According to International Patent Classification (IPC) or to both national classification and IPC

B. FIELDS SEARCHED

Minimum documentation searched (classification system followed by classification symbols)
Int.Cl$^6$ C07D213/00-89, C07D239/00-26, C09K19/00-42, G02F1/00-13

Documentation searched other than minimum documentation to the extent that such documents are included in the fields searched

Electronic data base consulted during the international search (name of data base and, where practicable, search terms used)
CAPLUS (STN), REGISTRY (STN)

C. DOCUMENTS CONSIDERED TO BE RELEVANT

| Category* | Citation of document, with indication, where appropriate, of the relevant passages | Relevant to claim No. |
|---|---|---|
| A | WO, 96/11897, A1 (Chisso Corp.), 25 April, 1996 (25. 04. 96), Refer to the full text & EP, 786445, A1 | 1-11 |
| A | JP, 8-325173, A (Chisso Corp.), 10 December, 1996 (10. 12. 96), Refer to the full text & WO, 96/37451, A1 & EP, 828700, A1 | 1-11 |

☐ Further documents are listed in the continuation of Box C. ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

Date of the actual completion of the international search
8 September, 1998 (08. 09. 98)

Date of mailing of the international search report
22 September, 1998 (22. 09. 98)

Name and mailing address of the ISA/
Japanese Patent Office

Facsimile No.

Authorized officer

Telephone No.

Form PCT/ISA/210 (second sheet) (July 1992)